# EUROPEAN PATENT APPLICATION

(11) **EP 2 008 658 A1**
(43) Date of publication of application: **31.12.2008**
(21) Application number: 07075536.8
(22) Date of filing: 28.06.2007
(51) Int. Cl.: A61K 31/4439, A61K 31/4406, A61K 45/06, A61P 35/00, A61P 27/06, A61P 13/12, A61P 1/16, A61P 9/10, A61P 25/00

(54) **Synergistic combination of anthranilamide pyridinureas and benzamide derivatives**

(71) Applicant: Bayer Schering Pharma Aktiengesellschaft, 13353 Berlin (DE)
(72) Inventor: Hess-Stumpp, Holger, 13503 Berlin (DE)

(57) **Abstract**

Pharmaceutical combinations comprising as compound A at least one compound from the group of angiogenesis inhibitors of general formula I 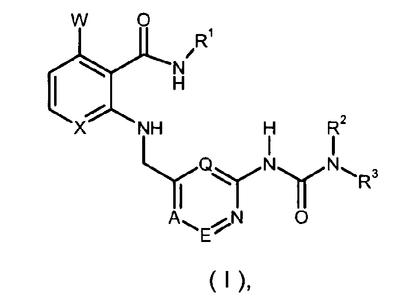 and as compound B at least one compound from the group of histone deacetylase inhibitors (HDAC) of general formula II 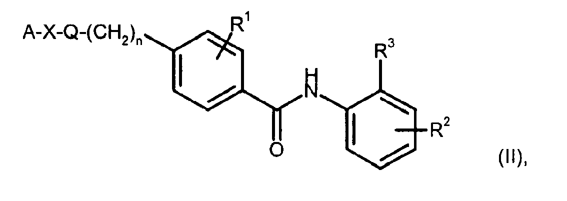 and their use for the treatment of different diseases resulting by persistent angiogenesis, are described.

## Description

The invention concerns a synergistically pharmaceutical combination and the use of said combination for the treatment of diseases resulting from persistent angiogenesis.
The synergistically inventive combination comprises an angiogenesis-inhibitor together with a histonedeacetylase (HDAC)-inhibitor.

Persistent angiogenesis can be the source for different diseases, such as for example psoriasis, arthritis, such as rheumatoid arthritis, haemeangioma, angiofribroma, diseases of the eyes, such as diabetic retinopathie, neovascular glaucoma, diseases of the kidney, such as glomerulonephritis, diabetic nephropatic desease, malignant nephrosclerosis, thrombotic microangiopatic syndrome, disposes of transplants and glomerulopathy, fibrotic diseases, such as liver cirrhosis, mesangial cell proliferative diseases and artheriosclerosis, or can be change for the worse of these diseases.

A direct or indirect inhibition of the VEGF-receptor can be used for the treatment of the described diseases and other VEGF-induced pathological angiogenesis and vascular permeable conditions, such as tumor vasculature.
For example, it is known that the growth of a tumor can be inhibited by soluble receptors and antibodies against VEGF.

Persistent angiogenesis can be induced by VEGF via its receptor. For this, it is necessary that VEGF binds to the receptor and a tyrosine phosphorylation is achieved.

Compelling data implicate angiogenesis and tumor-associated neovascularization as a central step in the process of tumor growth, invasion, and metastasis. Angiogenesis involves multiple steps and pathways dependent on the local balance between positive and negative regulatory factors, as well as interactions among the tumor, its vasculature, and the surrounding extracellular tissue matrix. A tumor remains in a dormant state, where the cellular proliferation rate is balanced by the apoptotic rate, and it is unable to grow in size beyond a few millimeters if it has not acquired an angiogenic phenotype.

VEGF-A, an endothelial cell specific mitogen, is considered to play a key role in angiogenic processes apparent in tumor growth. It has been shown to be secreted by hypoxic cells and cells of the reproductive apparatus under the regulation of oxygen partial pressure or hormones. VEGF has a variety of effects on vascular endothelium, including the ability to promote endothelial cell viability, mitogenesis, chemotaxis, and vascular permeability. It mediates its activity mainly via two tyrosine kinase receptors, VEGFR-1 (flt-1) and VEGFR-2 (flk-1/KDR), although other receptors, such as neuropilin-1 and -2, can also bind VEGF-A. Inhibition of VEGF-induced angiogenic signals will selectively target tumor-associated vessels, since cell division of endothelial cells in the normal vasculature is a very rare event and those cells are in a stabilized environment with pericytes and smooth muscle cells that render them stable in the absence of VEGF. Therefore, antiangiogenic therapy through inhibition of VEGF-mediated effects, is expected to be safe and well tolerated in cancer patients. VEGF-A is also a potent inducer of vascular permeability (second name: vascular permeability factor, VPF) and may also play a key role in ascitic fluid formation and oedema associated with malignant disease. Two VEGF-analogs, VEGF-C and VEGF-D have been described that bind to VEGFR-2 and VEGFR-3. The latter receptor appears to be responsible for lymphangiogenesis and may play a role in lymphogenic metastasis.
A VEGF signal inhibitor will not directly inhibit tumor cell growth. It will influence tumor growth by inhibiting tumor vascularization. It needs a constant long term application to exert its efficacy. The desired compound should therefore not cause major adverse effects that compromise the patients quality of life.

VEGF signal inhibitors are intended for a continuous long lasting therapy. It is clear that VEGF signal inhibitors are much better tolerated than conventional cytotoxic antitumor agents if they are specific.
VEGF signal inhibitors will interfere with important physiological processes (wound healing, menstrual cycle, pregnancy, fetal development) which may impose treatment interruptions and restrictions of indications. At present, none of these questions appears to compromise patient treatment. This is due to the fact that the majority of patients passed the reproductive age. Healing of small wounds might be regulated through other pathways like FGF signaling. From animal experiments and from treatments of patients it is known that potential effects on hematopoetic and other stem cells that express VEGFR did not materialize in changes of blood cell composition. VEGFR occurring in glomeruli of the kidney and in one cell layer near the chorioid plexus did not react with recognizable functional deficits on kinase blockade.
The instant invention especially concerns the treatment of cancer, melanomas and tumors.

In WO 2006/048251 anthranilamide pyridinurea derivatives are described which show angiogenesis inhibiting activity. These compounds of the general formula I 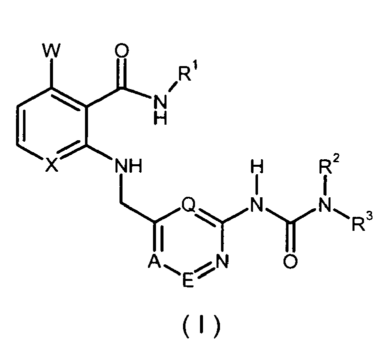 wherein
- X: is CH or N, preferably CH;
- W: is hydrogen or fluorine; preferably hydrogen;
- A, E and Q: independently of one another, are CH or N, whereby only a maximum of two nitrogen atoms are contained in the ring; preferably A, E, and Q are each CH;
- R¹: is aryl or heteroaryl, which may be optionally substituted in one or more places in the same way or differently with halogen, hydroxy, C₁-C₁₂-alkyl, C₂-C₆-alkenyl, C₁-C₁₂-alkoxy, halo-C₁-C₆-alkyl, =O, -SO₂R⁶, -OR⁵, -SOR⁴, -COR⁶, -CO₂R⁶ or -NR⁷R⁸, whereby C₁-C₁₂-alkyl may be substituted with -OR⁵ or -NR⁷R⁸, with the proviso that when R² and R³ are both -CH₃, R¹ is not any one of the following: 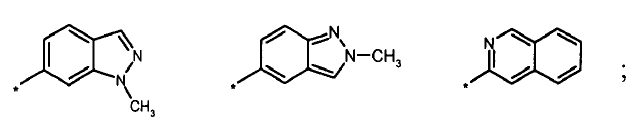 preferably heteroaryl optionally substituted in one or more places in the same way or differently with halogen, hydroxy, C₁-C₁₂-alkyl, C₂-C₆-alkenyl, C₁-C₁₂-alkoxy, halo-C₁-C₆-alkyl, =O, -SO₂R⁶, -OR⁵, -SOR⁴, -COR⁶, -CO₂R⁶ or -NR⁷R⁸, whereby C₁-C₁₂-alkyl may be substituted with -OR⁵ or -NR⁷R⁸, with the proviso that when R² and R³ are both -CH₃, R¹ is not any one of the following : 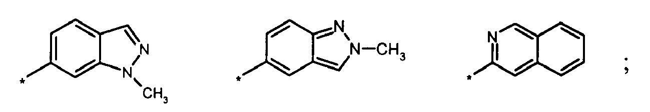 more preferably heteroaryl substituted in one or more places in the same way or differently with halogen, hydroxy, C₁-C₁₂-alkyl, C₂-C₆-alkenyl, C₁-C₁₂-alkoxy, halo-C₁-C₆-alkyl, =O, -SO₂R⁶, -OR⁵, -SOR⁴, -COR⁶, -CO₂R⁶ or -NR⁷R⁸, whereby C₁-C₁₂-alkyl may be substituted with -OR⁵ or -NR⁷R⁸, with the proviso that when R² and R³ are both -CH₃, R¹ is not any one of the following: 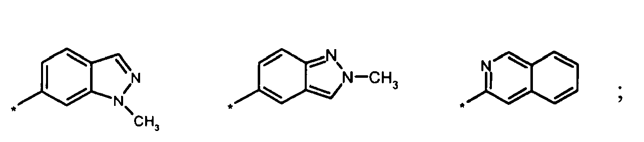 even more preferably R¹ is 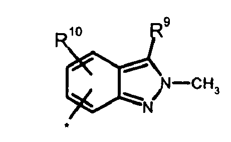 wherein R⁹ is hydrogen, halogen, C₁-C₁₂-alkyl, C₁-C₁₂-alkoxy, halo-C₁-C₆-alkyl, -COR⁶, -CO₂R⁶ or -NR⁷R⁸, whereby C₁-C₁₂-alkyl may be substituted with -OR⁵ or -NR⁷R⁸ and R¹⁰ is hydrogen or halogen; preferably R⁹ is hydrogen and R¹⁰ is hydrogen or halogen,
preferably fluorine; more particularly preferably R⁹ and R¹⁰ are both hydrogen;
more particularly preferably R¹ is 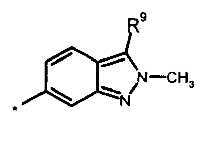 wherein R⁹ is hydrogen, halogen, C₁-C₁₂-alkyl, C₁-C₁₂-alkoxy, halo-C₁-C₆-alkyl, -COR⁶, -CO₂R⁶ or -NR⁷R⁸, whereby C₁-C₁₂-alkyl may be substituted with -OR⁵ or -NR⁷R⁸; more particularly preferably R⁹ is hydrogen;
- R² and R³,: independently of one another, are C₁-C₁₂ alkyl optionally substituted with -OR⁵; preferably C₁-C₂ alkyl optionally substituted with -OR⁵; more preferably unsubstituted C₁-C₂ alkyl; more particularly preferably are both -CH₃;
- R⁴: is C₁-C₁₂-alkyl, C₃-C₈-cycloalkyl, aryl or heteroaryl; preferably C₁-C₁₂-alkyl; more particularly preferably -CH₃;
- R⁵: is hydrogen, C₁-C₁₂-alkyl, C₃-C₈-cycloalkyl or halo-C₁-C₆-alkyl; preferably -CH₃ or hydrogen; more particularly preferably hydrogen;
- R⁶: is hydrogen, C₁-C₁₂-alkyl, C₃-C₈-cycloalkyl, halo-C₁-C₆-alkyl, aryl,
- or: -NR⁷R⁸; preferably C₁-C₁₂-alkyl or -NR⁷R⁸; more particularly preferably -CH₃;
- R⁷ and R⁸,: independently of one another, are hydrogen, -SO₂R⁶, -COR⁶, aryl, C₃-C₈-cycloalkyl, C₁-C₁₂-alkyl, halo-C₁-C₁₂-alkyl, or C₁-C₁₂-alkoxy, whereby C₁-C₁₂-alkyl may be optionally substituted with -OR⁵ or -N(CH₃)₂, or R⁷ and R⁸ may also be chosen in such a way as to provide a 3-8 membered cycloalkyl ring, preferably a 4-7 membered cycloalkyl ring, more preferably a 5 or 6 membered cycloalkyl ring, which may optionally contain further heteroatoms, such as nitrogen, oxygen or sulphur, and may be optionally substituted in one or more positions in the same way or differently with halogen, cyano, C₁-C₁₂-alkyl, C₁-C₁₂-alkoxy, halo-C₁-C₆-alkyl, =O, -OR⁵ , COR⁶, -SR⁴, -SOR⁴ or -SO₂R⁶; preferably R⁷ and R⁸ independently of one another, are hydrogen, COR⁶, -SO₂R⁶, C₁-C₁₂-alkyl; more preferably hydrogen or C₁-C₁₂-alkyl; more particularly preferably hydrogen or -CH₃,
and as well as isomers, diastereoisomers, enantiomers, tautomers and salts thereof, are of interest as compound A) of the inventive synergistic combination and are herewith incorporated by reference.

As used herein, the term "alkyl" is defined in each case as a substituted or unsubstituted straight-chain or branched alkyl group, such as, for example, methyl, ethyl, propyl, isopropyl, butyl, isobutyl, tert-butyl, pentyl, isopentyl or hexyl, heptyl, octyl, nonyl, decyl, undecyl, or dodecyl.

As used herein, the term "alkoxy" is defined in each case as a straight-chain or branched alkoxy group, such as, for example, methyloxy, ethyloxy, propyloxy, isopropyloxy, butyloxy, isobutyloxy, tert-butyloxy, pentyloxy, isopentyloxy, hexyloxy, heptyloxy, octyloxy, nonyloxy, decyloxy, undecyloxy or dodecyloxy.

As used herein, the term "cycloalkyl" is defined as a monocyclic alkyl ring, such as cyclopropyl, cyclobutyl, cyclopentyl, cyclohexyl, or cycloheptyl, cyclooctyl, cyclononyl or cyclodecyl, and also as bicyclic rings or tricyclic rings, such as, for example, adamantanyl. The cycloalkyl group may also contain, one or more heteroatoms, such as oxygen, sulphur and/or nitrogen, such that a heterocycloalkyl ring is formed.

As used herein, the term "halogen" is defined in each case as fluorine, chlorine, bromine or iodine, with fluorine being preferred for compounds of formula (I-A).

As used herein, the term "halo-C₁-C₆-alkyl" is defined as a C₁-C₆ alkyl group wherein some or all hydrogen atoms are replaced by halogen atoms, preferably replaced with fluoro atoms. Preferred is the group CF₃.

As used herein, the term "alkenyl" is defined in each case as a straight-chain or branched alkenyl group that contains 2-6, preferably 2-4 carbon atoms. For example, the following groups can be mentioned: vinyl, propen-1-yl, propen-2-yl, but-1-en-1-yl, but-1-en-2-yl, but-2-en-1-yl, but-2-en-2-yl, 2-methyl-prop-2-en-1-yl, 2-methyl-prop-1-en-1-yl, but-1-en-3-yl, but-3-en-1-yl, and allyl.

As used herein, the term "aryl" is defined in each case has 6-12 carbon atoms, such as, for example, cyclopropenyl, cyclopentadienyl, phenyl, tropyl, cyclooctadienyl, indenyl, naphthyl, azulenyl, biphenyl, fluorenyl, anthracenyl etc, phenyl being preferred.

As used herein, the term "C₁-C₁₂", as used throughout this text e.g. in the context of the definitions of "C₁-C₁₂-alkyl" and "C₁-C₁₂-alkoxy", is to be understood as meaning an alkyl or alkoxy group having a finite number of carbon atoms of 1 to 12, i.e. 1, 2, 3, 4, 5, 6, 7, 8, 9, 10, 11 or 12 carbon atoms. It is to be understood further that said term "C₁-C₁₂" is to be interpreted as any subrange comprised therein, e.g. C₁-C₁₂ , C₂-C₁₁, C₃-C_{10,} C₄-C₉, C₅-C₈, C₆-C₇, C₁-C₂. C₁-C₃, C₁-C₄ , C₁-C₅, C₁-C₆, C₁-C₇, C₁-C₈, C₁-C₉, C₁-C₁₀, C₁-C_{11 ;} preferably C₁-C₂. C₁-C₃, C₁-C₄. C₁-C₅, C₁-C_{6;} more preferably C₁-C₃.

Similarly, as used herein, the term "C₂-C₆", as used throughout this text e.g. in the context of the definitions of "C₂-C₆-alkenyl", is to be understood as meaning an alkenyl group having a finite number of carbon atoms of 2 to 6, i.e. 2, 3, 4, 5, or 6 carbon atoms. It is to be understood further that said term "C₂-C₆" is to be interpreted as any subrange comprised therein, e.g. C₂-C₆, C₃-C₅ , C₃-C₄, C₂-C₃ , C₂-C₄ , C₂-C_{5 ;} preferably C₂-C₃ .

Further as used herein, the term "C₁-C₆", as used throughout this text e.g. in the context of the definitions of "halo-C₁-C₆-alkyl" , is to be understood as meaning a haloalkyl group having a finite number of carbon atoms of 1 to 6, i.e. 1, 2, 3, 4, 5, or 6 carbon atoms. It is to be understood further that said term "C₁-C₆" is to be interpreted as any subrange comprised therein, e.g. C₁-C₆, C₂-C₅, C₃-C₄ , C₁ -C₂ , C₁-C₃ , C₁-C₄, C₁-C₅, C₁-C₆; more preferably C₁-C₃.

As used herein, the term "heteroaryl" as defined in each case, is an aromatic ring system which contains, in the ring, at least one heteroatom which may be identical or different, and which comprises 3-16 ring atoms, preferably 5 or 6 atoms, more preferably 9 or 10 ring atoms, said heteroatom being such as oxygen, nitrogen or sulphur, and can be monocyclic, bicyclic, or tricyclic, and in addition in each case can be benzocondensed.
Preferably heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, etc., and benzo derivatives thereof, such as, e.g., benzofuranyl, benzothienyl, benzoxazolyl, benzimidazolyl, benzotriazolyl, indazolyl, indolyl, isoindolyl, etc.; or pyridyl, pyridazinyl, pyrimidinyl, pyrazinyl, triazinyl, etc., and benzo derivatives thereof, such as, e.g., quinolinyl, isoquinolinyl, etc.; or azocinyl, indolizinyl, purinyl, etc., and benzo derivatives thereof; or cinnolinyl, phthalazinyl, quinazolinyl, quinoxalinyl, naphthpyridinyl, pteridinyl, carbazolyl, acridinyl, phenazinyl, phenothiazinyl, phenoxazinyl, xanthenyl, or oxepinyl, etc. More preferably the heteroaryl is selected from indazolyl, benzimidazolyl, quinolinyl, isoquinolinyl, benzotriazolyl.
Particularly preferably, the heteroaryl is indazolyl.

In principle, all these compounds show activity in different tumor indications when analysing the results of preclinical experiments. However, the results show that tumors are existing which show only a pour activity when these compounds are used in mono therapies. These results confirm the knowledge in oncology that only in few cases a monotherapy is ufficient in patients. A known exception is, for example, Gleevec. Said compound is a kinase inhibitor which is used in CLL. As a rule, combinations of different active compounds are used in the tumor therapy, such as a combination of 5-fluoro uracil (5-FU), oxaliplatin and Leukovorin for the treatment of colorectal cancer. In a lot of cases, also these combinations show only a limited activity, which means they let to a timely restricted elongation of the survival. Further, it should be noted that for a lot of tumor deseases, such as the melanom, approved combinations with chemotherapeutica are not existing. Therefore, there is still a big and urgend demand for active suitable combinations for a better therapy of tumors which are difficult to treat.

A preferred heteroaryl in position R¹ of general formula I is 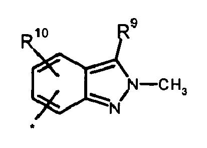 wherein R⁹ is hydrogen, halogen, C₁-C₁₂-alkyl, C₁-C₁₂-alkoxy, halo-C₁-C₆-alkyl, -COR⁶, -CO₂R⁶ or -NR⁷R⁸, whereby C₁-C₁₂-alkyl may be substituted with -OR⁵ or -NR⁷R⁸ and R¹⁰ is hydrogen or halogen; preferably R⁹ is hydrogen and R¹⁰ is hydrogen or halogen, preferably fluorine; more particularly preferably R⁹ and R¹⁰ are both hydrogen;
more particularly preferably R¹ is 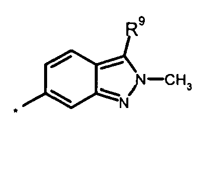 wherein R⁹ is hydrogen, halogen, C₁-C₁₂-alkyl, C₁-C₁₂-alkoxy, halo-C₁-C₆-alkyl, -COR⁶, -CO₂R⁶ or -NR⁷R⁸, whereby C₁-C₁₂-alkyl may be substituted with -OR⁵ or -NR⁷R⁸; more particularly preferably R⁹ is hydrogen;

The aryl group and the heteroaryl group in each case can be substituted in the same way or differently in one or more positions with halogen, hydroxy, C₁-C₁₂-alkyl, C₂-C₆-alkenyl, C₁-C₁₂-alkoxy, halo-C₁-C₆-alkyl, =O, -SO₂R⁶, -OR⁵, -SOR⁴, -COR⁶, -CO₂R⁶ or -NR⁷R⁸, whereby C₁-C₁₂-alkyl may be substituted with -OR⁵ or -NR⁷R⁸.
It is understood that the substitution on the aryl group and the heteroaryl group may take place on any one of the group's carbon atoms and/or on any one of the heteroatoms.

Preferably, the aryl group and the heteroaryl group is substituted in one or two positions.

If an acid group is included, the physiologically compatible salts of organic and inorganic bases are suitable as salts, such as, for example, the readily soluble alkali salts and alkaline-earth salts as well as N-methyl-glucamine, dimethyl-glucamine, ethyl-glucamine, lysine, 1,6-hexadiamine, ethanolamine, glucosamine, sarcosine, serinol, tris-hydroxy-methyl-amino-methane, aminopropanediol, Sovak base, and 1-amino-2,3,4-butanetriol.

If a basic group is included, the physiologically compatible salts of organic and inorganic acids are suitable, such as hydrochloric acid, sulphuric acid, phosphoric acid, citric acid, tartaric acid, succinic acid, fumaric acid, etc.

The compounds of general formula (I) according to the invention also contain the possible tautomeric forms and comprise the E-isomers or Z-isomers, or, if one or more stereogenic centers are present, racemates and/or enantiomers and/or diastereoisomers. Thus, a molecule with a single stereogenic center may be a mixture of enantiomers (R,S), or may be a single (R) or (S) enantiomer. A molecule with more than one stereogenic centre may be a mixture of diastereoisomers, or may be a single diastereoisomer, whereby the diastereoisomers may also exist as mixtures of enantiomers or single enantiomers.

It has now been found that a combination of an angiogenesis inhibitor (compound A) together with an HDAC-Inhibitor (compound B) results in a better activity against tumors, in comparison to the monotherapy of both inhibitor classes.

On the other hand, histone modification, catalized by the histone acetyltansferase (HAT) and histone deacetylase (HDAC) enzymes, plays an important role in regulating gene expression by altering chromatin structure. Histone acetylation results in charge neutralization and separation of DNA from the histones. Thus, transcriptionally activated genes are typically associated with hyperacetylated loci. Because of the profound effect of histone modification on gene transcription, manipulation of the activities of histone-modifing HAT and HDAC enzymes has the potential for modifing the cell cycle and the neoplastic transfomation of cells. Some compounds with deacetylase inhibitory activity have been shown to be effective in suppressing tumor growth in animal models and in initial clinical trials, although the efficacy is limited, possibly due to their stability, low retention, or nonspecific toxicity in the body.

In addition, HDAC enzymes play a role in the regulation of hypoxia-induced angiogenesis. Hypoxia enhances HDAC function and HDAC is closely involved in angiogenesis through suppression of hypoxia-responsive tumor suppressor genes. A specific HDAC inhibitor upregulates p53 and von Hippel-Lindau expression and downregulates hypoxia-induced factor-1 alpha and vascular endothelial growth factor (VEGF). HDAC inhibitors are also shown to inhibit angiogenesis both in vitro and in vivo.

HDAC inhibitors are currently of major interest as potential anti-cancer therapeutics, largely because of their well-documented properties of inhibiting proliferation and induce apotosis of tumour cells. Furthermore, the finding that HDAC appears to be a critical regulator of angiogenesis in addition to tumour cell growth will cause further interest in the development of HDAC inhibitors as potential anticancer drugs.

In EP 0847992 (US 6,174,905) benzamide derivatives are described which show differentiation-inducing effects, and which are useful a therapeutic or improving agent for malignant tumors, autoimmune diseases, dermatologic diseases and parasitism. In particular, they are highly effective as an anticancer drug, specifically to a hematologic malignancy and a solid carcinoma.

These compounds of general formula II 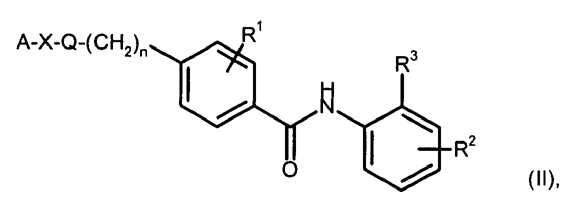 wherein
A is an optionally substituted phenyl group or an optionally substituted heterocyclic group wherein the substituent(s) for the phenyl group or the heterocyclic group is (are) 1 to 4 substituents selected from the group consisting of a halogen atom, a hydroxyl group, an amino group, a nitro group, a cyano group, an alkyl group having 1 to 4 carbons, an alkoxy group having 1 to 4 carbons, an aminoalkyl group having 1 to 4 carbons, an alkylamino group having 1 to 4 carbons, an acyl group having 1 to 4 carbons, an acylamino group having 1 to 4 carbons, an alkylthio group having 1 to 4 carbons, a perfluoroalkyl group having 1 to 4 carbons, a perfluoroalkyloxy group having 1 to 4 carbons, a carboxyl group, an alkoxycarbonyl group having 1 to 4 carbons, a phenyl group and a heterocyclic group;
X is a bond or a moiety having the following structure 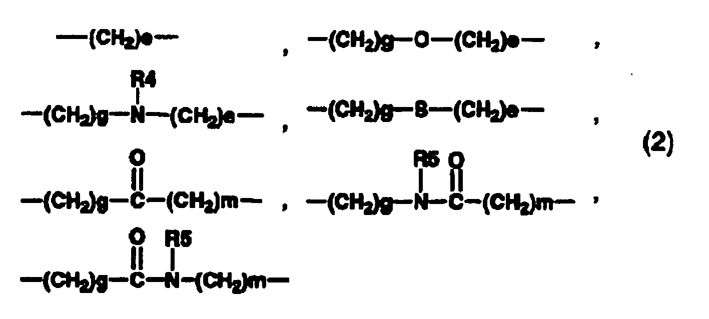 wherein e is an integer of 1 to 4; g and m are independently an integer of 0 to 4; R⁴ is a hydrogen atom or an optionally substituted alkyl group having 1 to 4 carbons, or the acyl group represented by formula (3) 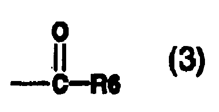 wherein R⁶ is an optionally substituted alkyl group having 1 to 4 carbons, a perfluoroalkyl group having 1 to 4 carbons, a phenyl group or a heterocyclic group; R⁵ is a hydrogen atom or an optionally substituted alkyl group having 1 to 4 carbons;
n is an integer of 0 to 4, provided that when X is a bond, n is not zero;
Q is a moiety having a structure selected from those illustrated in formula (4) 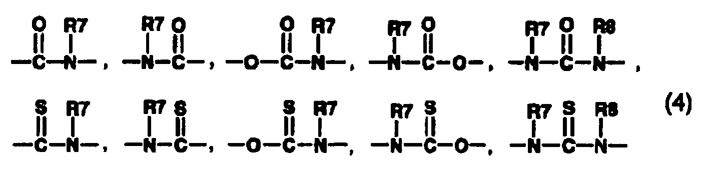 wherein R⁷ and R⁸ are independently a hydrogen atom or an optionally substituted alkyl group having 1 to 4 carbons;
R¹ and R² are independently a hydrogen atom, a halogen atom, a hydroxyl group, an amino group, an alkyl group having 1 to 4 carbons, an alkoxy group having 1 to 4 carbons, an aminoalkyl group having 1 to 4 carbons, an alkylamino group having 1 to 4 carbons, an acyl group having 1 to 4 carbons, an acylamino group having 1 to 4 carbons, an alkylthio group having 1 to 4 carbons, a perfluoroalkyl group having 1 to 4 carbons, a perfluoroalkyloxy group having 1 to 4 carbons, a carboxyl group or an alkoxycarbonyl group having 1 to 4 carbons; and
R³ is a hydroxyl or amino group or a pharmaceutically acceptable salt thereof, are of interest as compound B) of the inventive synergistic combination.

Of special interest are those compounds of formula II), wherein n is an integer of 1 to 4.

Of further interest are those compounds wherein Q is selected from the structures illustrated in formula (5): 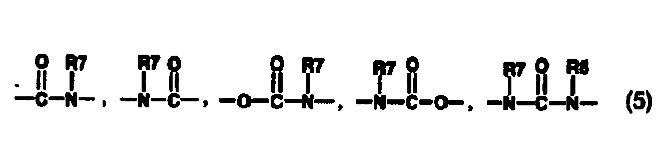 wherein R7 and R8 are as defined above.

Of further interest are also those compounds wherein A is an optionally substituted hetero ring, especially an optionally substituted pyridyl group.

Of special interest are also those compounds of general formula II), wherein n is 1 to 4; Q is selected from the structures illustrated in formula (5); A is an optionally substituted hetero ring, especially optionally substituted pyridyl group; most preferred, wherein X is direct bond, most preferred wherein R¹ and R² are a hydrogen atom, most preferred, wherein R³ is an amino group.

Of special interest are also those compounds of general formula II), wherein n is 1 to 4; wherein Q is selected from the structures illustrated in formula (5); A is an optionally substituted hetero ring, especially optionally substituted pyridyl group; most preferred, wherein X is the structure represented by formula (6):

-(CH₂)e- (6)

wherein e is an integer of 1 to 4; most preferred wherein n is 1 and R¹ and R² are a hydrogen atom; most preferred, wherein R³ is an amino group.

Of special interest are also those compounds of general formula II), wherein Q is selected from the structures illustrated in formula (5); A is an optionally substituted hetero ring, especially optionally substituted pyridyl group; most preferred,
wherein X is selected from the structures illustrated in formula (7):
wherein e, g and R4 are as defined above; most preferred wherein n is 1 and R¹ 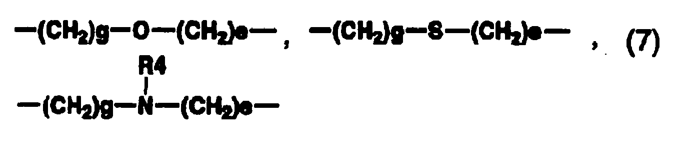 and R² are a hydrogen atom; most preferred, wherein R³ is an amino group.

Of special interest are also those compounds of general formula II, wherein Q is selected from the structures illustrated in formula (5); A is an optionally substituted hetero ring, especially optionally substituted pyridyl group; most preferred,
wherein X is selected from the structures illustrated in formula (8): 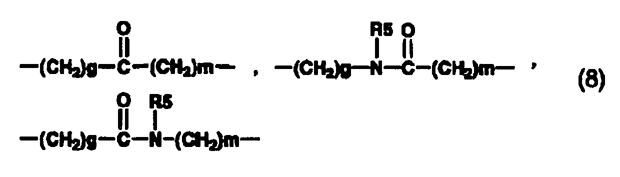 wherein g, m and R5 are as defined above; most preferred wherein n is 1 and R¹ and R² are a hydrogen atom; most preferred, wherein R³ is an amino group.

Of special interest are also those compounds of general formula II), wherein n is zero, and further of interest, wherein Q is selected from the structures illustrated in formula (5), and further of interest, wherein A is an optionally substituted hetero ring, most preferred, wherein A is anoptionally substituted pyridyl group, most preferred, wherein R¹ and R² are a hydrogen atom, most preferred, wherein R³ is an amino group.

In the above formula (II), n maybe zero or an integer of 1 to 4.
Q in the above formula (II) may be any structure illustrated in formula (5); 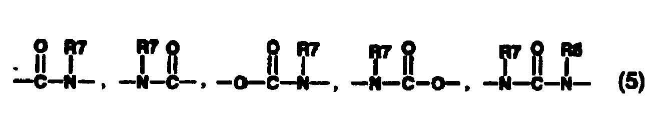 wherein R7 and R8 are as defined above.

X in the above formula (II) may be a moiety having the structure represented by formula (6);

-(CH₂)ₑ- (6)

wherein e is as defined above.

X in the above formula (II) may be also a moiety having any structure illustrated in formula (7); 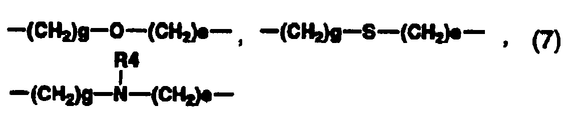 wherein e, g and R⁴ are as defined above.

X in the above formula (II) may be also a moiety having any structure illustrated in formula (8); 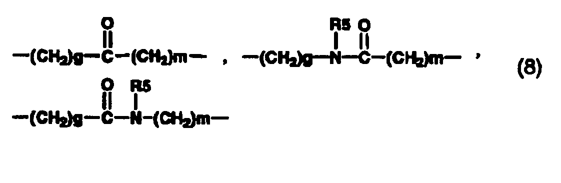 wherein g, m and R⁵ are as defined above.

As used herein, "1 to 4 carbons" means a carbon number per a single substituent; for example, for dialkyl substitution it means 2 to 8 carbons.

A heterocycle in the compound represented by formula (II) is a monocyclic heterocycle having 5 or 6 members containing 1 to 4 nitrogen, oxygen or sulfur atoms or a bicyclic-fused heterocycle. The monocyclic heterocycle includes pyridine, pyrazine, pyrimidine, pyridazine, thiophene, furan, pyrrole, pyrazole, isoxazole, isothiazole, imidazole, oxazole, thiazole, piperidine, piperazine, pyrrolidine, quinuclidine, tetrahydrofuran, morpholine, thiomorpholine and the like. The bicyclic fused heterocycle includes quinoline; isoquinoline; naphthyridine; fused pyridines such as furopyridine, thienopyridine, pyrrolopyridine, oxazolopyridine, imidazolopyridine and thiazolopyridine; benzofuran; benzothiophene; benzimidazole and the like.

A halogen may be fluorine, chlorine, bromine or iodine.

An alkyl having 1 to 4 carbons includes methyl, ethyl, n-propyl, isopropyl, n-butyl, isobutyl, sec-butyl and tert-butyl.

An alkoxy having 1 to 4 carbons includes methoxy, ethoxy, n-propoxy, isopropoxy, allyloxy, n-butoxy, isobutoxy, sec-butoxy, tert-butoxy and the like.

An aminoalkyl having 1 to 4 carbons includes aminomethyl, 1-aminoethyl, 2-aminopropyl and the like.

An alkylamino having 1 to 4 carbons includes N-methylamino, N,N-dimethylamino, N,N-diethylamino, N-methyl-N-ethylamino, N,N-diisopropylamino and the like.

An acyl having 1 to 4 carbons includes acetyl, propanoyl, butanoyl and like.

An acylamino having 1 to 4 carbons includes acetylamino, propanoylamino, butanoylamino and the like.

An alkylthio having 1 to 4 carbons includes methylthio, ethylthio, propylthio and the like.

A perfluoroalkyl having 1 to 4 carbons includes trifluoromethyl, pentafluoroethyl and the like.

A perfluoroalkyloxy having 1 to 4 carbons includes trifluoromethoxy, pentafluoroethoxy and the like.

An alkoxycarbonyl having 1 to 4 carbons includes methoxycarbonyl and ethoxycarbonyl.

An optionally substituted alkyl having 1 to 4 carbons includes methyl, ethyl, n-propyl, isopropyl, n-butyl, isobutyl, sec-butyl and tert-butyl and these having 1 to 4 substituents selected from the group consisting of a halogen, hydroxyl, amino, nitro, cyano, phenyl and a heterocycle.

A basic cyclic structure includes cyclic moieties having 4 to 7 members containing carbons and/or hetero atoms or their fused cycles. For example it may be cyclobutane, cyclopentane, cyclohexane, cycloheptane, oxetane, oxolane, oxane, oxepane, pyrrolidine, imidazolidine, pyrazolidine, piperidine, piperazine, indoline, isoindoline, thiolane, thiazolidine and oxazolidine rings, which may contain unsaturated bonds, hydrogen bond acceptors and/or substituents.

In the above analyses, other commercially available program packages such as CATALYST(MSI), Cerius 2/QSAR+(MSI) and SYBYL/DISCO(Tripos) may be used, and the information on distance obtained here is not limited to that from a particular calculation program.

The ring centroid used in definition of the spatial configuration may be defined as an average of X, Y and Z axes of the ring-forming atoms. When a ring structure to be calculated is fused-polycyclic, the centroid of either the overall fused ring or of a partial ring may be used as that for defining the space.

Selected compounds of general formula II are for example the following compounds: 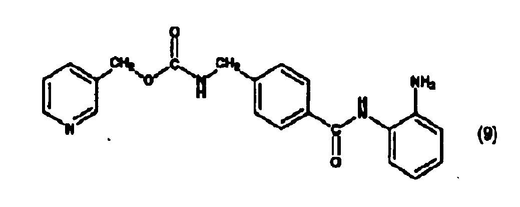 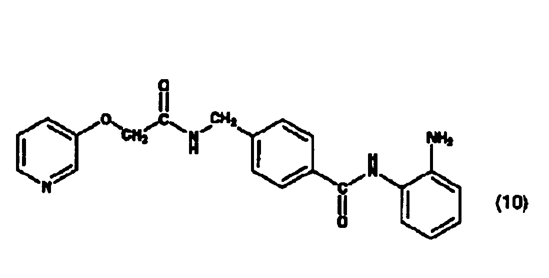 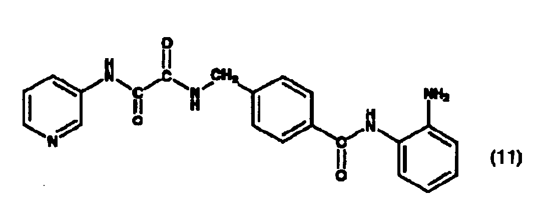 and

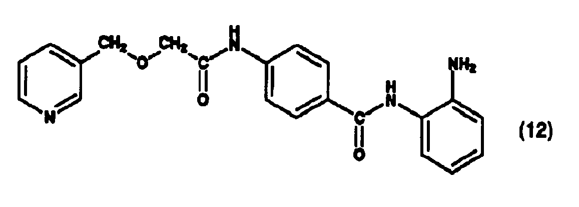

"Possibility of formation of a configuration" means that a conformer filling the spatial configuration is within 15 kcal/mol, preferably 8 kcal/mol from the energetically most stable structure.

Specific calculation can be performed as described in the instructions for Sybyl (M.Clark) or J.Comput.Chem. 10, 982(1989).

A pharmaceutically acceptable salt of the compound of this invention includes salts with an inorganic acid such as hydrochloric acid, hydrobromic acid, sulfuric acid and phosphoric acid; and with an organic acid such as acetic acid, lactic acid tartaric acid, malic acid, succinic acid, fumaric acid, maleic acid, citric acid, benzoic acid, trifluroacetic acid, p-toluenesulfonic acid and methanesulfonic acid. Such a salt includes
N-(2-aminophenyl)-4-[N-(pyridin-3-yl)methoxycarbonylaminomethyl]benzamide hydrochloride,
N-(2-aminophenyl)-4-[N-(pyridin-3-yl)methoxycarbonylaminomethyl]benzamideh ydrobromide,
N-(2-aminophenyl)-4-[N-(pyridin-3-yl)methoxycarbonylaminomethyl]benzamide sulfate,
N-(2-aminophenyl)-4-[N-(pyridin-3-yl)methoxycarbonylaminomethyl]benzamide phosphate,
N-(2-aminophenyl)-4-[N-(pyridin-3-yl)methoxycarbonylaminomethyl]benzamide acetate,
N-(2-aminophenyl)-4-[N-(pyridin-3-yl)methoxycarbonylaminomethyl]benzamide lactate,
N-(2-aminophenyl)-4-[N-(pyridin-3-yl)methoxycarbonylaminomethyl]benzamide tartrate,
N-(2-aminophenyl)-4-[N-(pyridin-3-yl)methoxycarbonylaminomethyl]benzamide malate,
N-(2-aminophenyl)-4-[N-(pyridin-3-yl)methoxycarbonylaminomethyl]benzamide succinate,
N-(2-aminophenyl)-4-[N-(pyridin-3-yl)methoxycarbonylaminomethyl]benzamide fumarate,
N-(2-aminophenyl)-4-[N-(pyridin-3-yl)methoxycarbonylaminomethyl]benzamide maleate,
N-(2-aminophenyl)-4-[N-(pyridin-3-yl)methoxycarbonylaminomethyl]benzamide citrate,
N-(2-aminophenyl)-4-[N-(pyridin-3-yl)methoxycarbonylaminomethyl]benzamide trifluoroacetate,
N-(2-aminophenyl)-4-[N-(pyridin-3-yl)methoxycarbonylaminomethyl]benzamide p-toluenesulfonate and
N-(2-aminophenyl)-4-[N-(pyridin-3-yl)methoxycarbonylaminomethyl]benzamide methanesulfonate.

When having asymmetric carbon or carbons, the compound represented by formula (II) may be obtained as an individual stereoisomer or a mixture of stereoisomers including a racemic modification. This invention encompasses the above-specified different forms, which may be also used as an active ingredient.

Representative compounds B) represented by formula (II) are specifically shown in Tables 1 to 4, but this invention is not intended to be limited to these.

**TABLE 1**

| | | | | | | | |
|---|---|---|---|---|---|---|---|
| 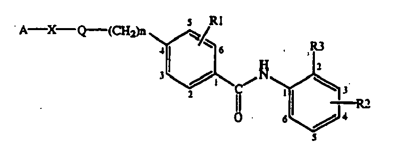 | | | | | | | |

| Compound No. | A | X | Q | n | R1 | R2 | R3 |
|---|---|---|---|---|---|---|---|
| 1 | 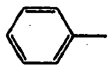 | Direct bond | 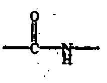 | 1 | H | H | NH₂ |
| 2 | 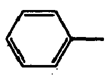 | -CH₂- | 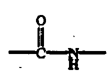 | 0 | H | H | NH₃ |
| 3 | 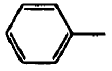 | -(CH₂)₂- | 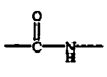 | 0 | H | H | NH₂ |
| 4 | 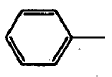 | -(CH₂)₃- | 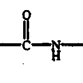 | 0 | H | H | NH₂ |
| 5 | 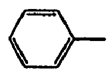 | -(CH₃)₄- | 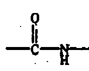 | 0 | H | H | NH₂ |
| 6 | 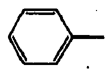 | -CH₂- | 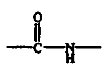 | 1 | H | H | NH₂ |
| 7 | 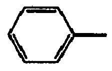 | -(CH₂)₂- | 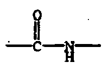 | 1 | H | H | NH₂ |
| 8 | 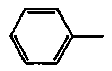 | -CH₃- | 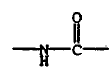 | 0 | H | H | NH₂ |
| 9 | 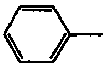 | -(CH₂)₂- | 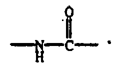 | 0 | H | H | NH₂ |
| 10 | 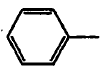 | Direct bond | 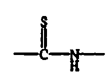 | 1 | H | H | NH₂ |
| 11 | 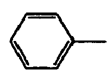 | -CH₃- | 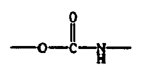 | 1 | H | H | NH₂ |
| 12 | 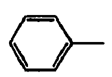 | Direct bond | 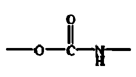 | 1 | H | H | NH₂ |
| 13 | 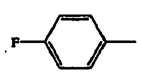 | Direct bond | 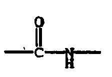 | 1 | H | H | NH₂ |
| 14 | 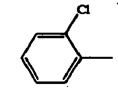 | Direct bond | 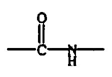 | 1 | H | H | NH₂ |
| 15 | 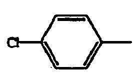 | -CH₂- | 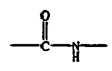 | 0 | H | H | NH₂ |
| 16 | 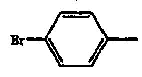 | Direct bond | 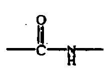 | 1 | H | H | NH₂ |
| 17 | 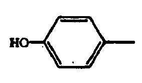 | Direct bond | 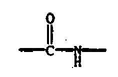 | 1 | H | H | NH₂ |
| 18 | 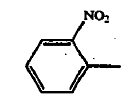 | Direct bond | 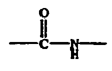 | 1 | H | H | NH₂ |
| 19 | 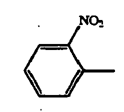 | -CH₂- | 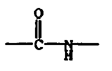 | 0 | H | H | NH₂ |
| 20 | 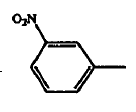 | Direct bond | 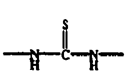 | 1 | H | H | NH₂ |
| 21 | 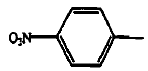 | -CH₂- | 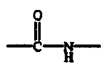 | 0 | H | H | NH₂ NH₂ |
| 22 | 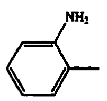 | -CH₂- | 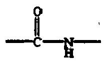 | 0 | H | H | NH₂ |
| 23 | 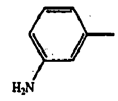 | -CH₂- | 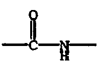 | 1 | H | H | NH₂ |
| 24 | 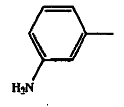 | Direct bond | 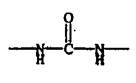 | 1 | H | H | NH₂ |
| 25 | 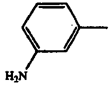 | Direct bond | 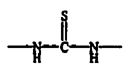 | 1 | H | H | NH₂ |
| 26 | 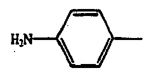 | -CH₂- | 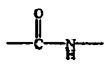 | 0 | H | H | NH₂ |
| 27 | 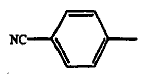 | Direct bond | 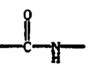 | 1 | H | H | NH₂ |
| 28 | 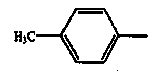 | Direct bond | 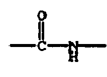 | 1 | H | H | NH₂ |
| 29 | 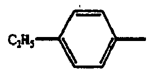 | Direct bond | 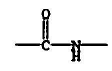 | 1 | H | H | NH₃ |
| 30 | 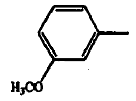 | Direct bond | 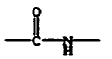 | 1 | H | H | NH₂ |
| 31 | 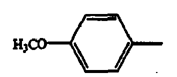 | Direct bond | 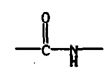 | 1 | H | H | NH₂ |
| 32 | 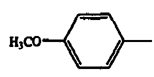 | -CH₂- | 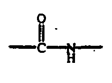 | 0 | H | H | NH₂ |
| 33 | 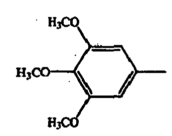 | Direct bond | 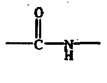 | 1 | H | H | NH₂ |
| 34 | 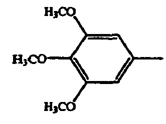 | -CH₂- | 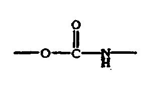 | 1 | H | H | NH₂ |
| 35 | 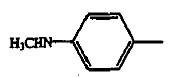 | Direct bond | 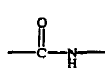 | 1 | H | H | NH₂ |
| 36 | 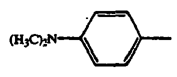 | Direct bond | 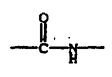 | 1 | H | H | NH₂ |
| 37 | 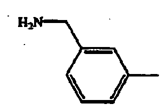 | Direct bond | 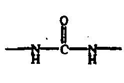 | 1 | H | H | NH₂ |
| 38 | 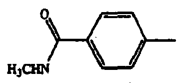 | -CH₂- | 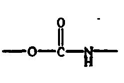 | 1 | H | H | NH₂ |
| 39 | 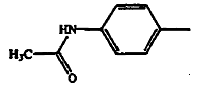 | -CH₂- | 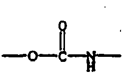 | 1 | H | H | NH₂ |
| 40 | 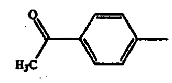 | Direct bond | 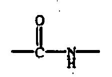 | 1 | H | H | NH₂ |
| 41 | 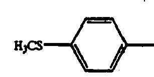 | Direct bond | 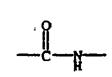 | 1 | H | H | NH₂ |
| 42 | 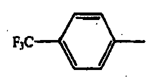 | Direct bond | 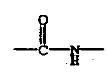 | 1 | H | H | NH₂ |
| 43 | 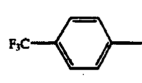 | -CH₂- | 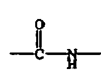 | 0 | H | H | NH₂ |
| 44 | 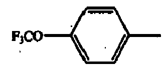 | Direct bond | 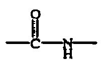 | 1 | H | H | NH₂ |
| 45 | 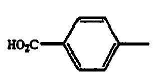 | Direct bond | 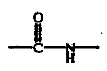 | 1 | H | H | NH₂ |
| 46 | 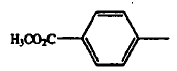 | Direct bond | 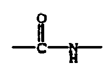 | 1 | H | H | NH₂ |
| 47 | 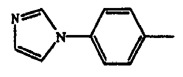 | -CH₂- | 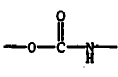 | 1 | H | H | NH₂ |
| 48 | 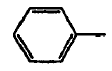 | -O-CH₂- | 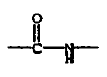 | 1 | H | H | NH₂ |
| 49 | 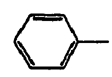 | -S-CH₂- | 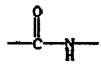 | 1 | H | H | NH₂ |
| 50 | 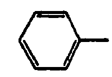 | 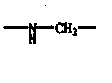 | 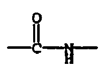 | 1 | H | H | NH₂ |
| 51 | 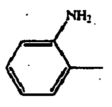 | -CH₂- | 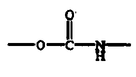 | 1 | H | H | NH₂ |
| 52 | 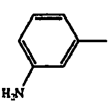 | -CH₂- | 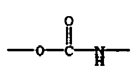 | 1 | H | H | NH₂ |
| 53 | 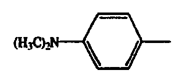 | -CH₂- | 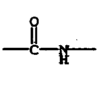 | 0 | H | H | NH₂ |
| 54 | 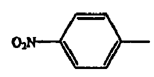 | -O-CH₂- | 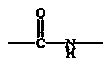 | 0 | H | H | NH₂ |
| 55 | 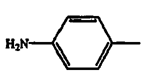 | -O-CH₂- | 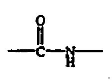 | 0 | H | H | NH₂ |
| 56 | 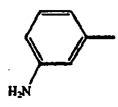 | -O-CH₂- | 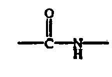 | 1 | H | H | NH₂ |
| 57 | 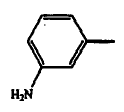 | -O-CH₂- | 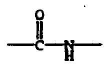 | 1 | H | 5-F | NH₂ |
| 58 | 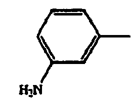 | -CH₂-O-CH₂- | 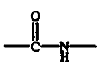 | 0 | H | H | NH₂ |
| 59 | 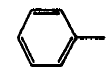 | 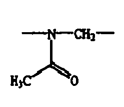 | 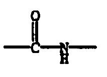 | 1 | H | H | NH₂ |
| 60 | 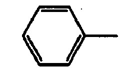 | 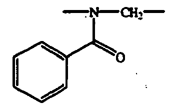 | 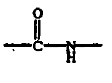 | 1 | H | H | KH₂ |
| 61 | 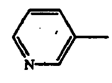 | -O-CH₂- | 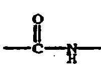 | 1 | H | H | NH₂ |
| 62 | 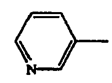 | -O-(CH₂)₂- | 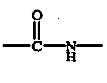 | 1 | H | H | NH₂ |
| 63 | 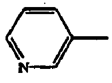 | 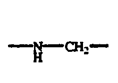 | 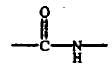 | 1 | H | H | NH₂ |
| 64 | 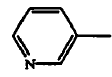 | -S-CH₂- | 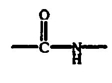 | 1 | H | H | NH₂ |
| 65 | 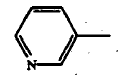 | -O-(CH₂)- | 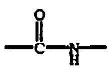 | 0 | H | H | NH₂ |
| 66 | 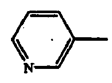 | -O-(CH₂)₂- | 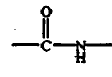 | 0 | H | H | NH₂ |
| 67 | 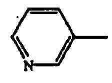 | -O-(CH₂)₂- | 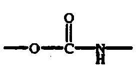 | 0 | H | H | NH₂ |
| 68 | 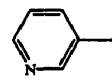 | -CH₂- | 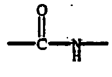 | 0 | H | H | NH₂ |
| 69 | 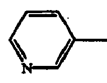 | -(CH₂)₂- | 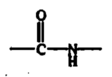 | 0 | H | H | NH₂ |
| 70 | 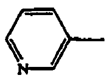 | -(CH₂)₂- | 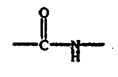 | 0 | H | H | NH₂ |
| 71 | 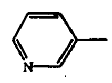 | Direct bond | 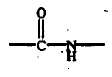 | 1 | H | H | NH₂ |
| 71 | 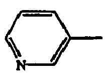 | Direct bond | 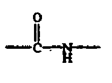 | 2 | H | H | NH₂ |
| 73 | 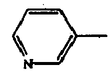 | Direct bond | 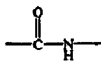 | 3 | H | H | NH₂ |
| 74 | 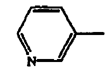 | -CH₂- | 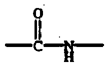 | 1 | H | H | NH₂ |
| 75 | 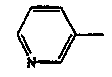 | -(CH₂)₂- | 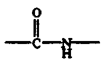 | 1 | H | H | NH₂ |
| 76 | 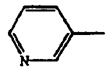 | -(CH₂)₃- | 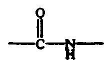 | 1 | H | H | NH₂ |
| 77 | 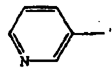 | -CH₂- | 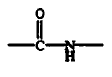 | 2 | H | H | NH₂ |
| 78 | 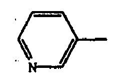 | -CH₂- | 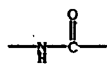 | 1 | H | H | NH₂ |
| 79. | 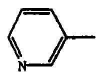 | Direct bond | 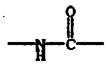 | 2 | H | H | NH₂ |
| 80 | 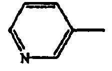 | -CH₂- | 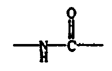 | 2 | H | H | NH₂ |
| 81 | 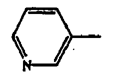 | Direct bond | 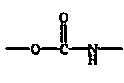 | 1 | H | H | NH₂ |
| 82 | 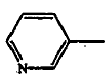 | -CH₂- | 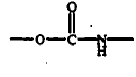 | 1 | H | H | NH₂ |
| 83 | 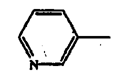 | -(CH₂)₂- | 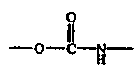 | 1 | H | H | NH₂ |
| 84 | 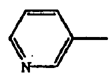 | -(CH₂)₃- | 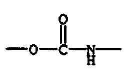 | 1 | H | H | NH₂ |
| 85 | 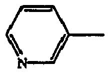 | -CH₂- | 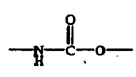 | 1 | H | H | NH₂ |
| 86 | 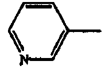 | -CH₂- | 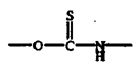 | 1 | H | H | NH₂ |
| 87 | 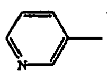 | Direct bond | 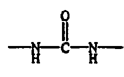 | 1 | H | H | NH₂ |
| 88 | 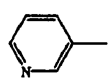 | -CH₂- | 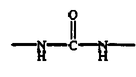 | 1 | H | H | NH₂ |
| 89 | 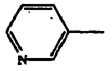 | -(CH₂)₂- | 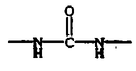 | 1 | H | H | NH₂ |
| 90 | 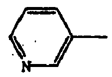 | -CH₂- | 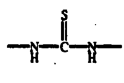 | 1 | H | H | NH₂ |
| 91 | 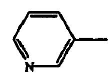 | -O-CH₂- | 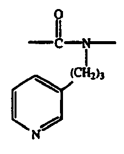 | 1 | H | H | NH₂ |
| 92 | 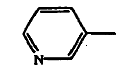 | -O-CH₂- | 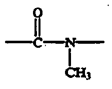 | 1 | H | H | NH₂⁻ |
| 93 | 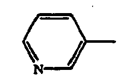 | -O-CH₂- | 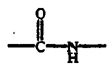 | 1 | H | H | OH |
| 94 | 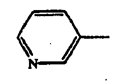 | 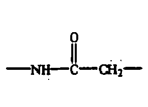 | 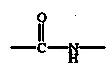 | 0 | H | H | NH₂ |
| 95 | 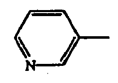 | 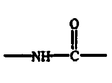 | 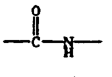 | 1 | H | H | NH₂ |
| 96 | 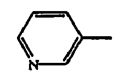 | 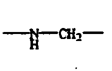 | 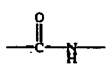 | 1 | H | H | NH₂ |
| 97 | 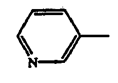 | 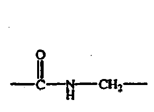 | 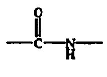 | 0 | H | H | NH₂ |
| 98 | 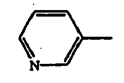 | 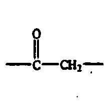 | 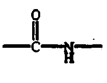 | 1 | H | H | NH₂ |
| 99 | 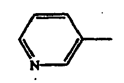 | 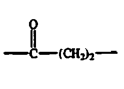 | 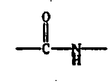 | 0 | H | H | NH₂ |
| 100 | 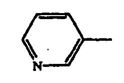 | 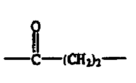 | 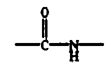 | 1 | H | H | NH₂ |
| 101 | 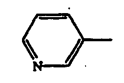 | -CH₃-O-CH₂- | 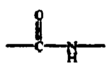 | 0 | H | H | NH₂ |
| 102 | 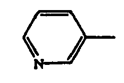 | -CH₂-O-CH₃- | 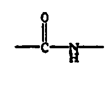 | 0 | 3-CH₃ | H | NH₂ |
| 103 | 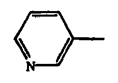 | -CH₂-O-CH₂- | 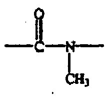 | 0 | H | H | NH₂ |
| 104 | 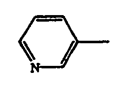 | 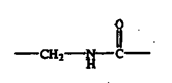 | 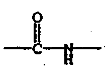 | 0 | H | H | NH₂ |
| 105 | 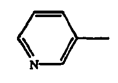 | 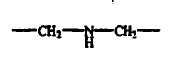 | 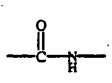 | 0 | H | H | NH₂ |
| 106 | 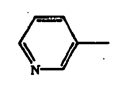 | 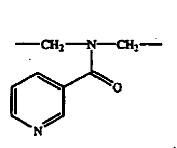 | 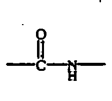 | 0 | H | H | NH₂ |
| 107 | 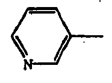 | 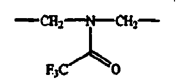 | 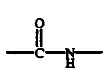 | 1 | H | H | NH₂ |
| 108 | 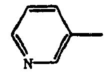 | 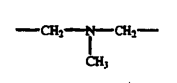 | 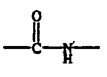 | 0 | H | H | NH₂ |
| 109 | 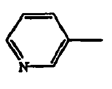 | -CH₂- | 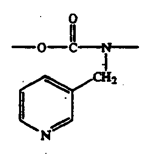 | 1 | H | H | NH₂ |
| 110 | 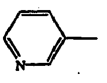 | -CH₂- | 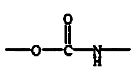 | 1 | H | 5-F | NH₂ |
| 111 | 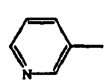 | -CH₂- | 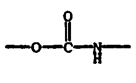 | 1 | H | H | OH |
| 112 | 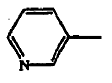 | -CH₂- | 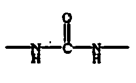 | 1 | H | 5-F | NH₂ |
| 113 | 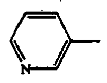 | -CH₂- | 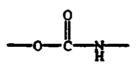 | 1 | H | 4-Cl | NH₂ |
| 114 | 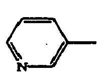 | -CH₂- | 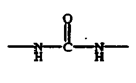 | 1 | H | H | OH |
| 115 | 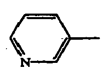 | -CH₂- | 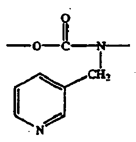 | 1 | H | H | OH |
| 116 | 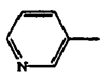 | -CH₂- | 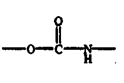 | 1 | H | 4-OH | OH |
| 117 | 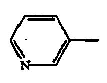 | -CH₂- | 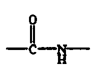 | 1 | H | H | OH |
| 118 | 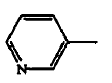 | -CH₂- | 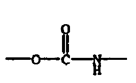 | 1 | H | 5-CH₃ | OH |
| 119 | 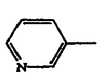 | -CH₃- | 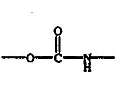 | 1 | H | 5-OCH₃ | OH |
| 120 | 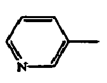 | -CH₂- | 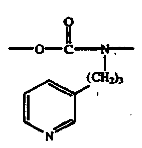 | 1 | H | H | NH₂ |
| 121 | 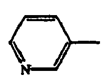 | -CH₂- | 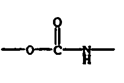 | 1 | H | 5-OCH₃ | NH₂ |
| 122 | 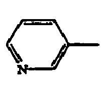 | -(CH₂)₃- | 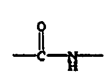 | 0 | H | 5-F | NH₂ |
| 123 | 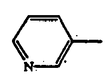 | -(CH₂)₂- | 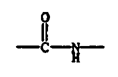 | 0 | 3-Cl | H | NH₂ |
| 124 | 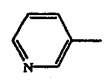 | -(CH₃)₂- | 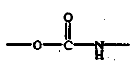 | 0 | H | H | NH₂ |
| 125 | 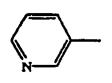 | -(CH₃)₂- | 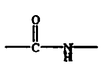 | 1 | H | H | OH |
| 126 | 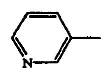 | 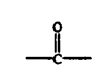 | 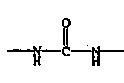 | 1 | H | H | NH₂ |
| 127 | 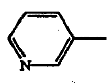 | 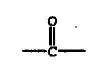 | 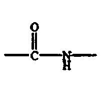 | 1 | H | H | NH₂ |
| 128 | 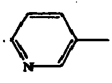 | -O-CH₂- | 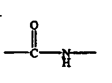 | 1 | 2-Cl | H | NH₂ |
| 129 | 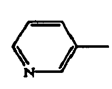 | -O-CH₂- | 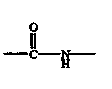 | 1 | H | 5-F | NH₂ |
| 130 | 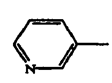 | -O-CH₂- | 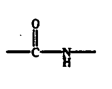 | 1 | H | 5-OCH₃ | NH₂ |
| 131 | 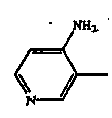 | -CH₂- | 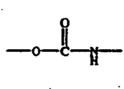 | 1 | H | H | NH₂ |
| 132 | 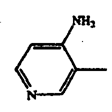 | -O-CH₂- | 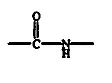 | 1 | H | H | NH₂ |
| 133 | 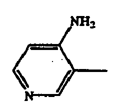 | -CH₂-O-CH₂- | 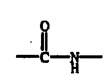 | 1 | H | H | NH₂ |
| 134 | 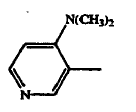 | -CH₂- | 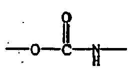 | 1 | H | H | NH₂ |
| 135 | 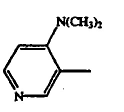 | -O-CH₂- | 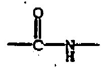 | 1 | H | H | NH₂ |
| 136 | 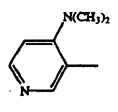 | -CH₂-O-CH₂- | 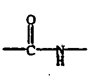 | 1 | H | H | NH₂ |
| 137 | 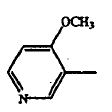 | -CH₂- | 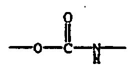 | 1 | H | H | NH₂ |
| 138 | 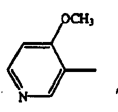 | -O-CH₂- | 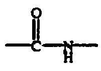 | 1 | H | H | NH₂ |
| 139 | 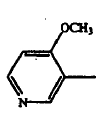 | -CH₂O-CH₂- | 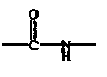 | 1 | H | H | NH₂ |
| 140 | 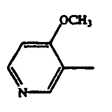 | -CH₂- | 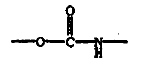 | 1 | H | 5-F | NH₂ |
| 141 | 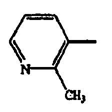 | Direct bond | 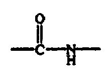 | 1 | H | H | NH₂ |
| 142 | 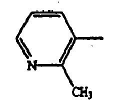 | -CH₂- | 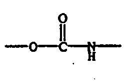 | 1 | H | H | NH₂ |
| 143 | 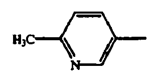 | Direct bond | 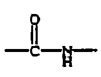 | 1 | H | H | NH₂ |
| 144 | 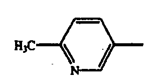 | -CH₂- | 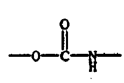 | 1 | H | H | NH₂ |
| 145 | 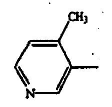 | -CH₂- | 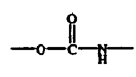 | 1 | H | H | NH₂ |
| 146 | 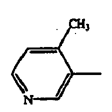 | -CH₂- | 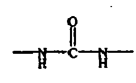 | 1 | H | H | NH₃ |
| 147 | 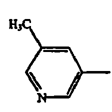 | -CH₂- | 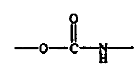 | 1 | H | H | NH₂ |
| 148 | 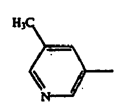 | -CH₂- | 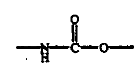 | 1 | H | H | NH₂ |
| 149 | 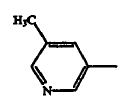 | -CH₂- | 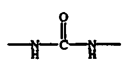 | 1 | H | H | NH₂ |
| 150 | 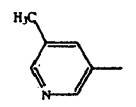 | -(CH₂)₂- | 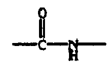 | 1 | H | H | NH₂ |
| 151 | 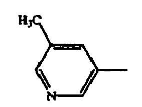 | -(CH₂)₂- | 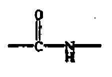 | 1 | H | H | NH₂ |
| 152 | 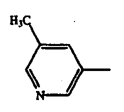 | -(CH₂)₂- | 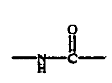 | 0 | H | H | NH₂ |
| 153 | 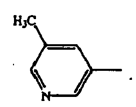 | -CH₂- | 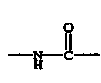 | 2 | H | H | NH₂ |
| 154 | 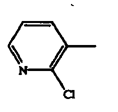 | Direct bond | 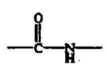 | 1 | H | H | NH₂ |
| 155 | 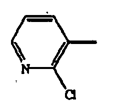 | -CH₂- | 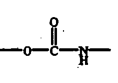 | 1 | H | H | NH₂ |
| 156 | 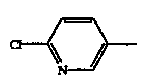 | Direct bond | 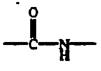 | 1 | H | H | NH₂ |
| 157 | 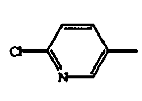 | -CH₂- | 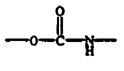 | 1 | H | H | NH₂ |
| 158 | 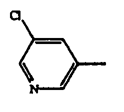 | -O-CH₂- | 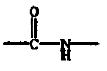 | 1 | H | H | NH₂ |
| 159 | 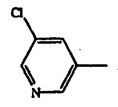 | -O-CH₂- | 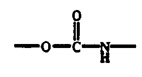 | 1 | H | H | NH₂ |
| 160 | 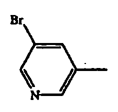 | -CH₂- | 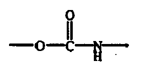 | 1 | H | H | NH₃ |
| 161 | 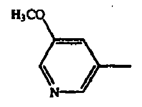 | -CH₂- | 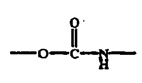 | 1 | H | H | NH₃ |
| 162 | 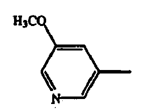 | -CH₂- | 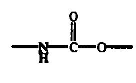 | 1 | H | H | NH₂ |
| 163 | 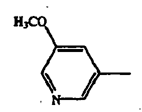 | -CH₂- | 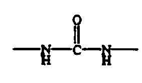 | 1 | H | H | NH₃ |
| 164 | 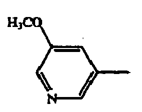 | -(CH₂)₂- | 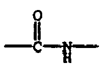 | 1 | H | H | NH₂ |
| 165 | 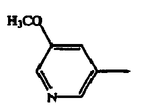 | -(CH₂)₂- | 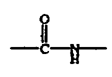 | 1 | H | H | NH₂ |
| 166 | 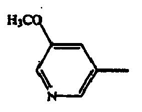 | -(CH₂)₂- | 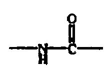 | 0 | H | H | NH₂ |
| 167 | 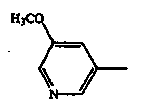 | -CH₂- | 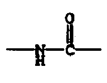 | 2 | H | H | NH₂ |
| 168 | 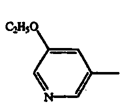 | -CH₂- | 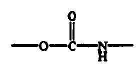 | 1 | H | H | NH₂ |
| 169 | 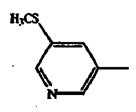 | -CH₂- | 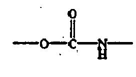 | 1 | H | H | NH₂ |
| 170 | 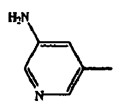 | -CH₂- | 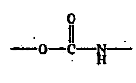 | 1 | H | H | NH₂ |
| 171 | 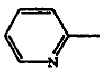 | -CH₂- | 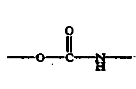 | 1 | H | H | NH₂ |
| 172 | 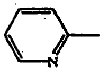 | -(CH₂)₂- | 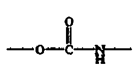 | 1 | H | H | NH₂ |
| 173 | 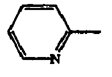 | Direct bond | 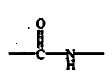 | 1 | H | H | NH₂ |
| 174 | 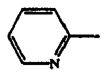 | -CH₂- | 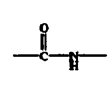 | 0 | H | H | NH₂ |
| 175 | 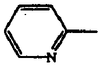 | -O-CH₂- | 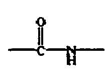 | 1 | H | 5-OCH₃ | NH₂ |
| 176 | 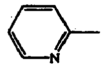 | -CH₂-O-CH₂- | 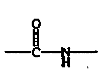 | 0 | H | H | NH₂ |
| 177 | 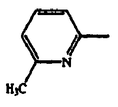 | -CH₂- | 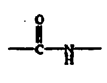 | 0 | H | H | NH₃ |
| 178 | 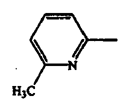 | Direct bond | 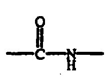 | 1 | H | H | NH₃ |
| 179 | 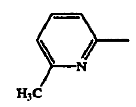 | -CH₂- | 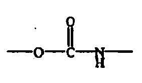 | 1 | H | H | NH₃ |
| 180 | 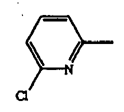 | -CH₂- | 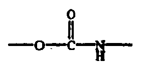 | 1 | H | H | NH₂ |
| 181 | 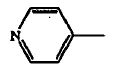 | -CH₂- | 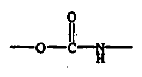 | 1 | H | H | NH₂ |
| 182 | 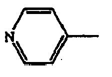 | -(CH₂)₂- | 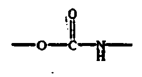 | 1 | H | H | NH₂ |
| 183 | 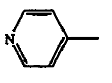 | Direct bond | 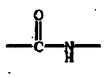 | 1 | H | H | NH₂ |
| 184 | 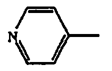 | -CH₂- | 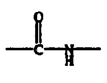 | 0 | H | H | NH2 |
| 185 | 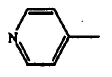 | -CH₂- | 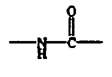 | 0 | H | H | NH₂ |
| 186 | 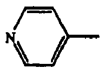 | -CH₂- | 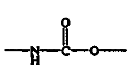 | 1 | H | H | NH₂ |
| 187 | 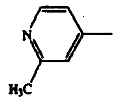 | -CH₂- | 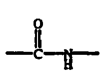 | 0 | H | H | NH₂ |
| 188 | 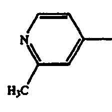 | Direct bond | 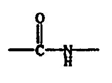 | 1 | H | H | NH₂ |
| 189 | 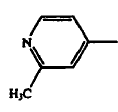 | -CH₂- | 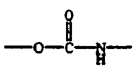 | 1 | H | H | NH₂ |
| 190 | 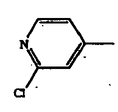 | -CH₂- | 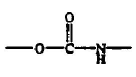 | 1 | H | H | NH₂ |
| 191 | 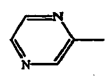 | Direct bond | 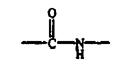 | 1 | H | H | NH₂ |
| 192 | 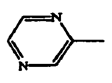 | -CH₃- | 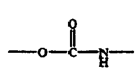 | 1 | H | H | NH₂ |
| 193 | 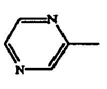 | -CH₂-O-CH₂- | 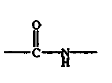 | 1 | H | H | NH₂ |
| 194 | 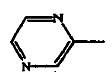 | | 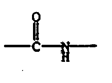 | 0 | H | H | NH₂ |
| 195 | 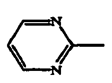 | Direct bond | 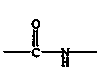 | 1 | H | H | NH₂ |
| 196 | 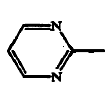 | -CH₂- | 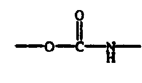 | 1 | H | H | NH₂ |
| 197 | 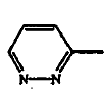 | Direct bond | 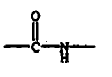 | 1 | H | H | NH₂ |
| 198 | 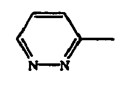 | -CH₂- | 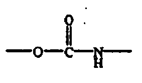 | 1 | H | H | NH₂ |
| 199 | 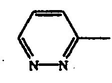 | -CH₂-O-CH₂- | 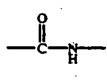 | 1 | H | H | NH₂ |
| 200 | 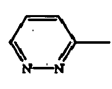 | -CH₂-O-CH₃- | 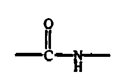 | 0 | H | H | NH₂ |
| 201 | 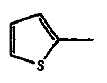 | Direct bond | 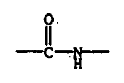 | 1 | H | H | NH₂ |
| 202 | 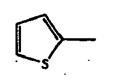 | -CH₂- | 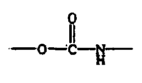 | 1 | H | H | NH₂ |
| 203 | 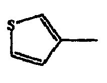 | -(CH₂)₂- | 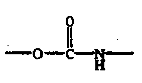 | 1 | H | H | NH₂ |
| 204 | 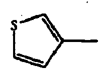 | -CH₂-O-CH₃ | 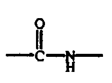 | 0 | H | H | NH₂ |
| 205 | 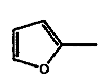 | Direct bond | 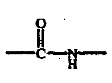 | 1 | H | H | NH₂ |
| 206 | 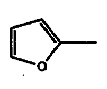 | -CH₂-O-CH₂- | 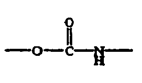 | 1 | H | H | NH₂ |
| 207 | 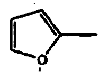 | | 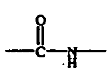 | 1 | H | H | NH₂ |
| 208 | 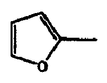 | CH₂-O-CH₂- | 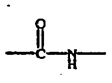 | 0 | H | H | KH₂ |
| 209 | 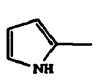 | Direct bond | 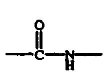 | 1 | H | H | NH₂ |
| 210 | 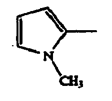 | Direct bond | 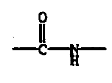 | 1 | H | H | NH₂ |
| 211 | 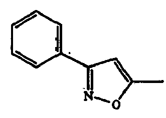 | -CH₂- | 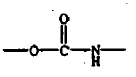 | 1 | H | H | NH₂ |
| 212 | 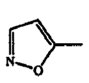 | Direct bond | 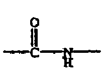 | 1 | H | H | NH₂ |
| 213 | 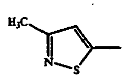 | Direct bond | 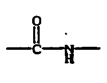 | 1 | H | H | NH₂ |
| 214 | 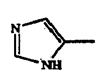 | Direct bond | 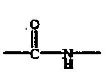 | 1 | H | H | NH₃ |
| 215 | 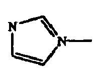 | -(CH₂)₃- | 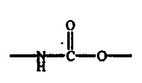 | 1 | H | H | NH₂ |
| 216 | 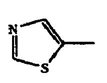 | -CH₂- | 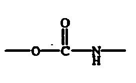 | 1 | H | H | NH₂ |
| 217 | 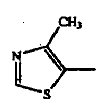 | -(CH₃)₂- | 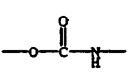 | 1 | H | H | NH₃ |
| 218 | 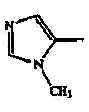 | -CH₂- | 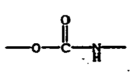 | 1 | H | H | NH₂ |
| 219 | 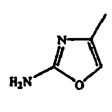 | -CH₂- | 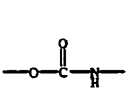 | 1 | H | H | NH₂ |
| 220 | 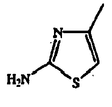 | -CH₂- | 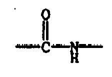 | 1 | H | H | NH₂ |
| 221 | 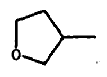 | -CH₂- | 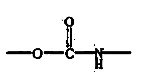 | 1 | H | H | NH₂ |
| 222 | 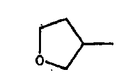 | -CH₂-O-CH₂- | 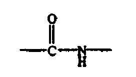 | 1 | H | H | NH₃ |
| 223 | 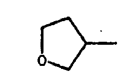 | -CH₂-O-CH₂- | 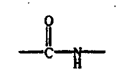 | 1 | H | H | NH₂ |
| 224 | 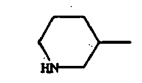 | Direct bond | 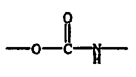 | 1 | H | H | NH₂ |
| 225 | 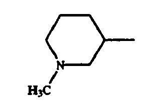 | -CH₂- | 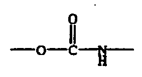 | 1 | H | H | NH₂ |
| 226 | 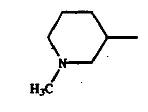 | -CH₂-O-CH₂- | 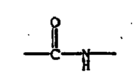 | 1 | H | H | NH₃ |
| 227 | 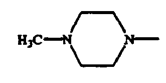 | -(CH₂)₃- | 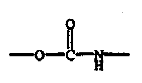 | 1 | H | H | NH₂ |
| 228 | 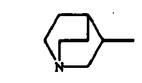 | Direct bond | 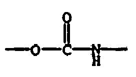 | 1 | H | H | NH₂ |
| 229 | 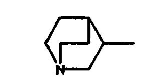 | -CH₂- | 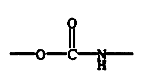 | 1 | H | H | NH₂ |
| 230 | 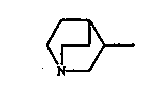 | -CH₂-O-CH₂- | 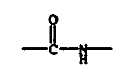 | 1 | H | H | NH₂ |

**TABLE 2**

| Compound No. | Structural formula |
|---|---|
| 1 | 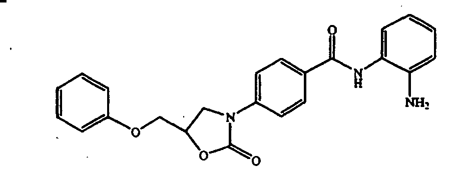 |
| 2 | 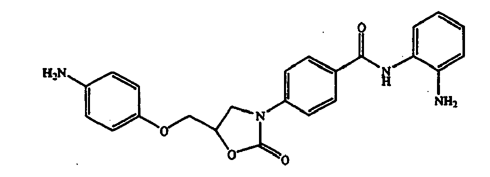 |
| 3 | 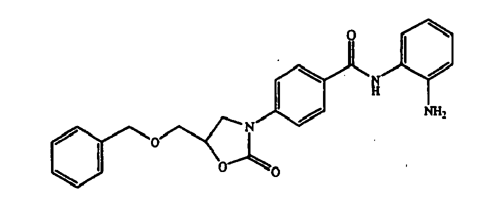 |
| 4 | 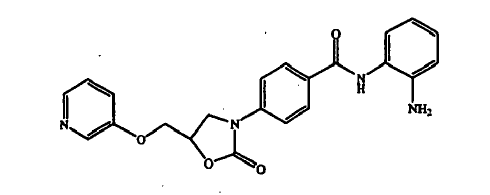 |
| 5 | 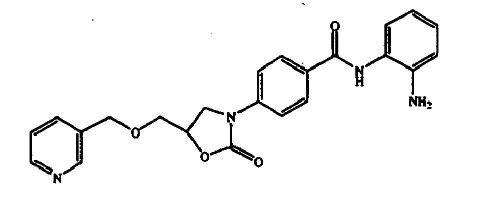 |
| 6 | 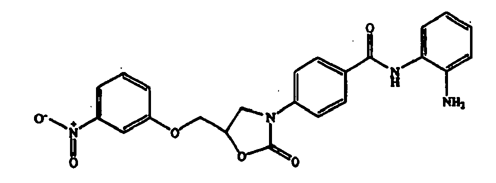 |
| 7 | 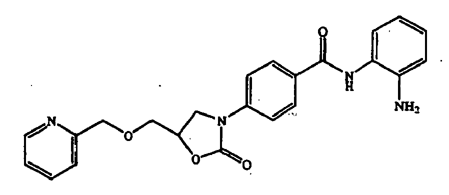 |
| 8 | 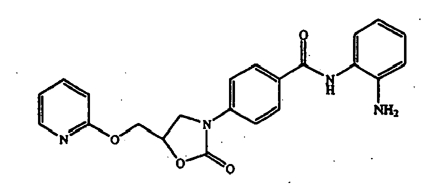 |
| 9 | 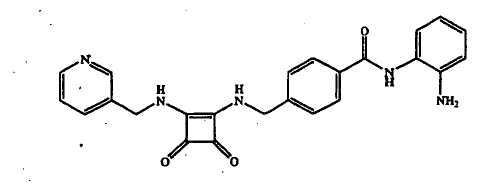 |
| 10 | 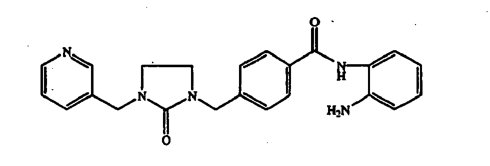 |
| 11 | 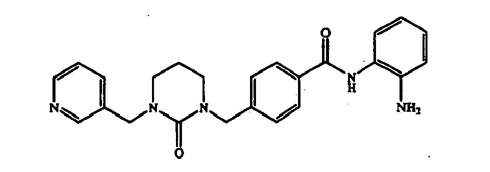 |
| 12 | 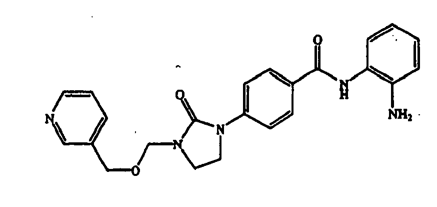 |
| 13 | 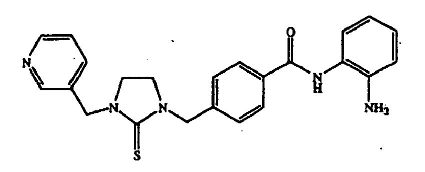 |
| 14 | 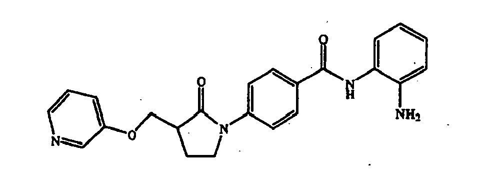 |
| 15 | 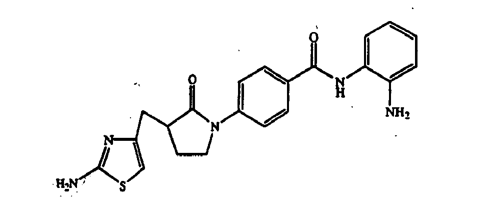 |
| 16 | 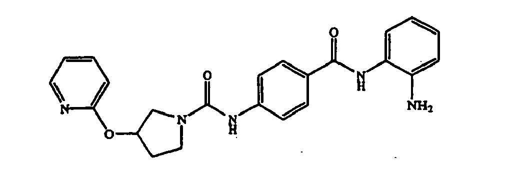 |
| 17 | 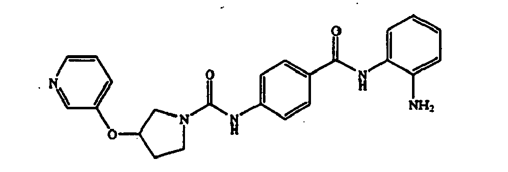 |
| 18 | 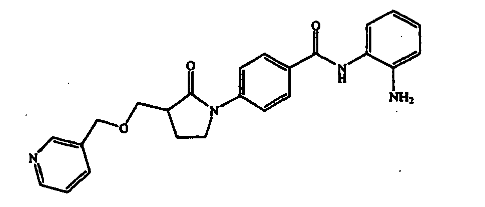 |
| 19 | 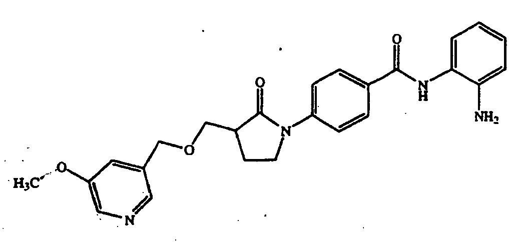 |
| 20 | 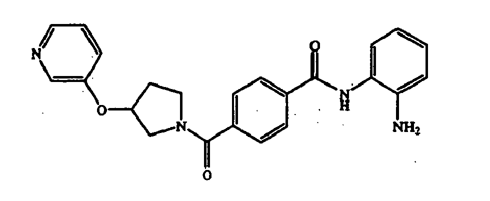 |

**Table 3**

| Compound No. | Structural formula |
|---|---|
| 5 | 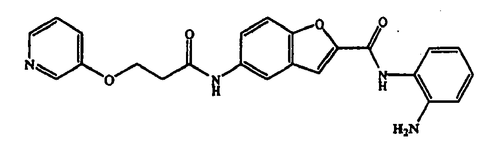 |
| 6 | 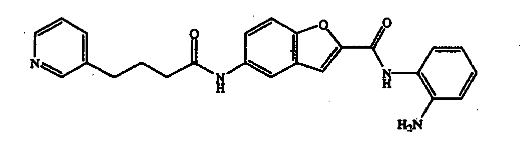 |
| 7 | 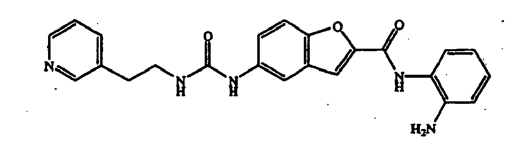 |
| 8 | 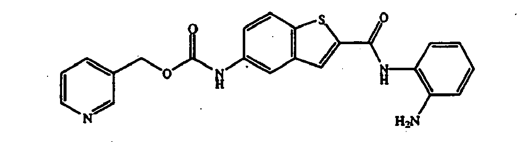 |
| 9 | 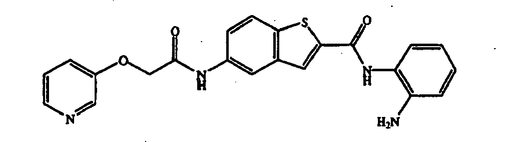 |
| 10 | 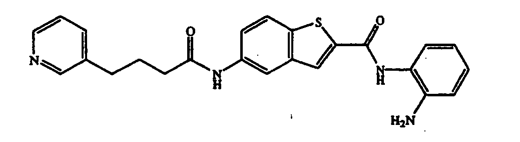 |
| 11 | 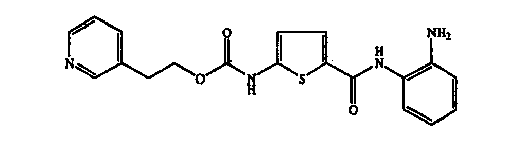 |
| 12 | 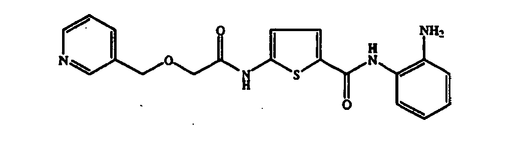 |
| 13 | 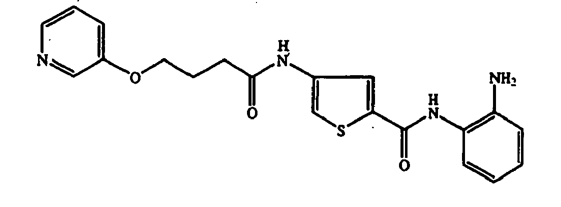 |
| 14 | 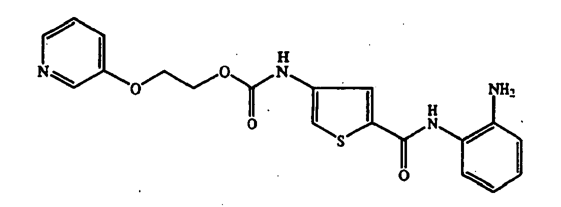 |
| 15 | 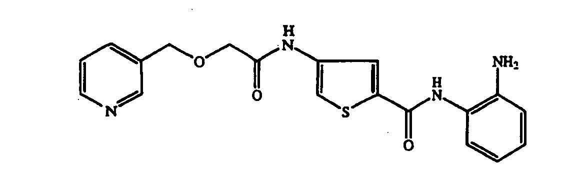 |
| 16 | 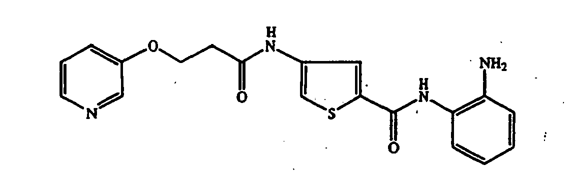 |

**TABLE 4**

| Compound No. | Structural formula |
|---|---|
| 1 | 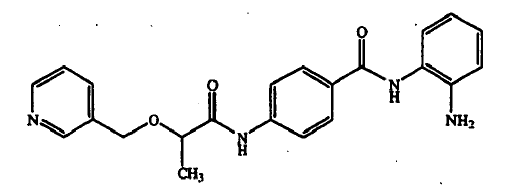 |
| 2 | 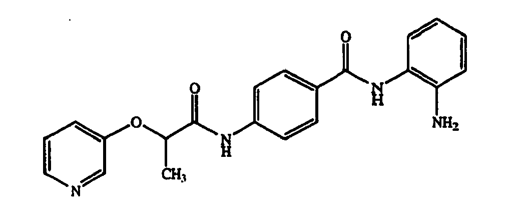 |
| 3 | 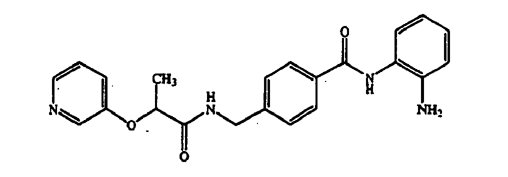 |
| 4 | 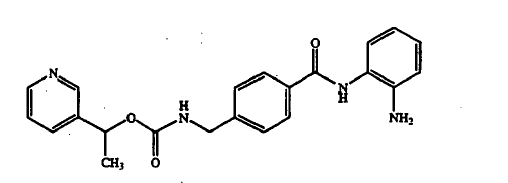 |
| 5 | 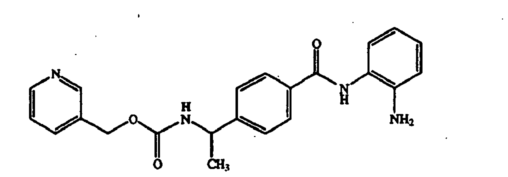 |
| 6 | 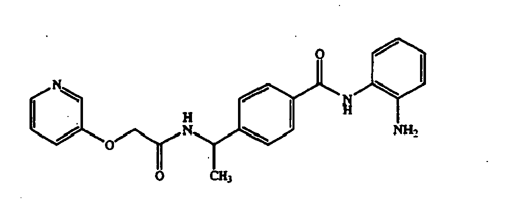 |
| 7 | 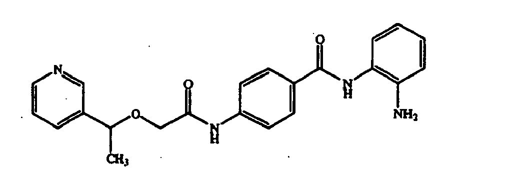 |
| 8 | 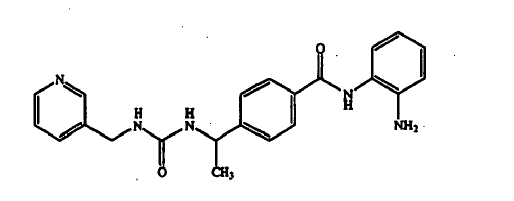 |
| 9 | 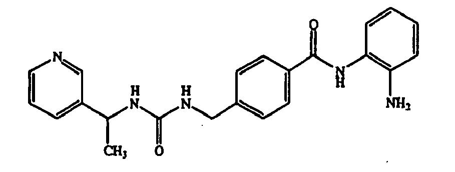 |
| 10 | 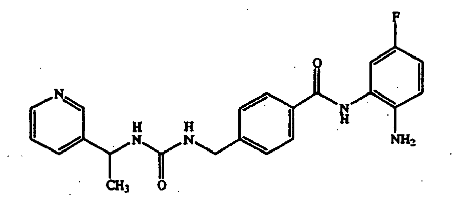 |
| 11 | 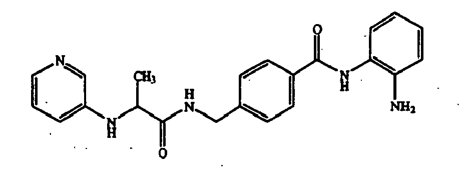 |
| 12 | 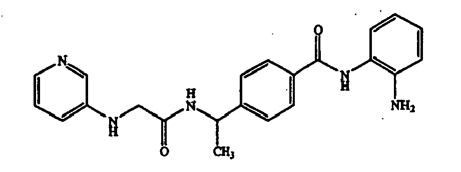 |
| 13 | 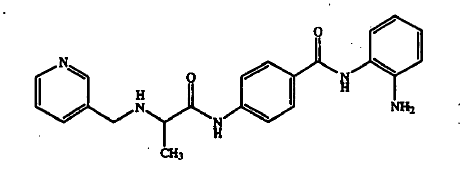 |
| 14 | 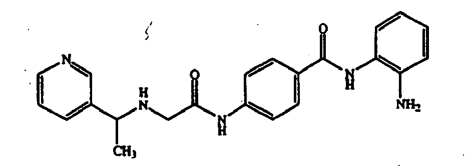 |

It should however be noted that these examples do not limit the inventive combination only to these compounds B.

The process for the production of the above mentioned compounds B is completely described in US 6,174,905.

Compounds described in WO 2006/048251 (compounds A) and compounds described in US 6,174,905) (compounds B) are incorporated by reference.

The compounds of WO 2006/048251 (compounds A) and compounds of US 6,174,905) (compounds B) show only minor effects on diseases, especially on tumors and melanoma if applied alone.

However, there is high demand for a medicament, or medicaments, which show a clear effect when applied to cancer or tumors, or diseases as discussed supra. Thus, there is a high demand for a medicament, such as a formulation or combination, respectively, which can overcome these problems.

It has now surprisingly been found that a combination comprising
a) as compound A
   at least one compound from the group compounds of general formula I 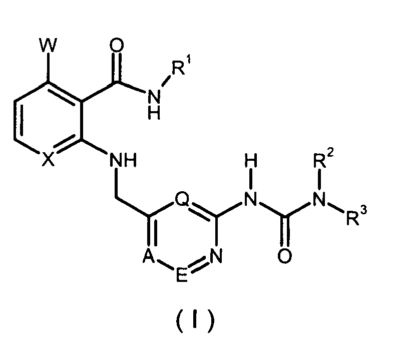 wherein
   - X: is CH or N;
   - W: is hydrogen or fluorine;
   - A, E and Q: independently of one another, are CH or N, whereby only a maximum of two nitrogen atoms are contained in the ring;
   - R¹: is aryl or heteroaryl, which may be optionally substituted in one or more places in the same way or differently with hydrogen, halogen, hydroxy, C₁-C₁₂-alkyl, C₂-C₆-alkenyl, C₁-C₁₂-alkoxy, halo-C₁-C₆-alkyl, =O, -SO₂R⁶, -OR⁵, -SOR⁴, -COR⁶, -CO₂R⁶ or -NR⁷R⁸, whereby C₁-C₁₂-alkyl may be substituted with -OR⁵ or -NR⁷R⁸;
   - R² and R³,: independently of one another, are C₁-C₁₂ alkyl optionally substituted with -OR⁵;
   - R⁴: is C₁-C₁₂-alkyl, C₃-C₈-cycloalkyl, aryl or heteroaryl;
   - R⁵: is hydrogen, C₁-C₁₂-alkyl, C₃-C₈-cycloalkyl or halo-C₁-C₆-alkyl;
   - R⁶: is hydrogen, C₁-C₁₂-alkyl, C₃-C₈-cycloalkyl, halo-C₁-C₆-alkyl, aryl, or -NR⁷R⁸;
   - R⁷ and R⁸,: independently of one another, are hydrogen, -SO₂R⁶, -COR⁶, aryl, C₃-C₈-cycloalkyl, C₁-C₁₂-alkyl, halo-C₁-C₁₂-alkyl, or C₁-C₁₂-alkoxy, whereby C₁-C₁₂-alkyl may be optionally substituted with -OR⁵ or -N(CH₃)₂, or R⁷ and R⁸ may also be chosen in such a way as to provide a 3-8 membered cycloalkyl ring, which may optionally contain further heteroatoms, such as nitrogen, oxygen or sulphur, and may be optionally substituted in one or more positions in the same way or differently with halogen, cyano, C₁-C₁₂-alkyl, C₁-C₁₂-alkoxy, halo-C₁-C₆-alkyl, =O, -OR⁵ , -COR⁶, -SR⁴, -SOR⁴ or -SO₂R⁶;
   and as well as isomers, diastereoisomers, enantiomers, tautomers and salts thereof,
   and
b) as compound B
   at least one compound from the group of compounds of formula II 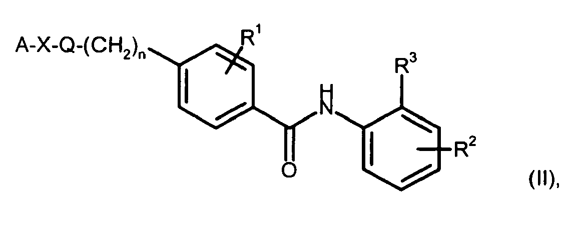
wherein
- A: is an optionally substituted phenyl group or an optionally substituted heterocyclic group wherein the substituent(s) for the phenyl group or the heterocyclic group is (are) 1 to 4 substituents selected from the group consisting of a halogen atom, a hydroxyl group, an amino group, a nitro group, a cyano group, an alkyl group having 1 to 4 carbons, an alkoxy group having 1 to 4 carbons, an aminoalkyl group having 1 to 4 carbons, an alkylamino group having 1 to 4 carbons, an acyl group having 1 to 4 carbons, an acylamino group having 1 to 4 carbons, an alkylthio group having 1 to 4 carbons, a perfluoroalkyl group having 1 to 4 carbons, a perfluoroalkyloxy group having 1 to 4 carbons, a carboxyl group, an alkoxycarbonyl group having 1 to 4 carbons, a phenyl group and a heterocyclic group;
X is a bond or a moiety having the following structure 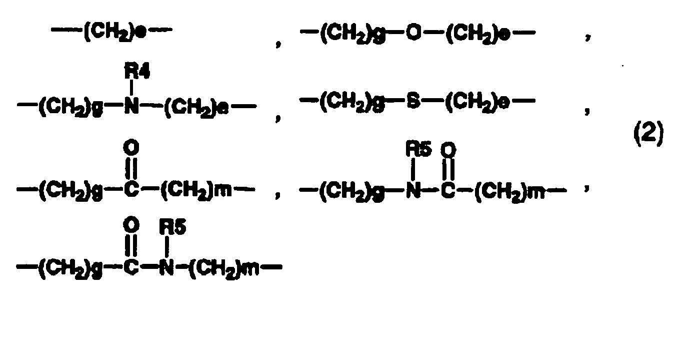 wherein e is an integer of 1 to 4; g and m are independently an integer of 0 to 4; R⁴ is a hydrogen atom or an optionally substituted alkyl group having 1 to 4 carbons, or the acyl group represented by formula (3) 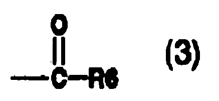 wherein R⁶ is an optionally substituted alkyl group having 1 to 4 carbons, a perfluoroalkyl group having 1 to 4 carbons, a phenyl group or a heterocyclic group; R⁵ is a hydrogen atom or an optionally substituted alkyl group having 1 to 4 carbons;
n is an integer of 0 to 4, provided that when X is a bond, n is not zero;
Q is a moiety having a structure selected from those illustrated in formula (4) 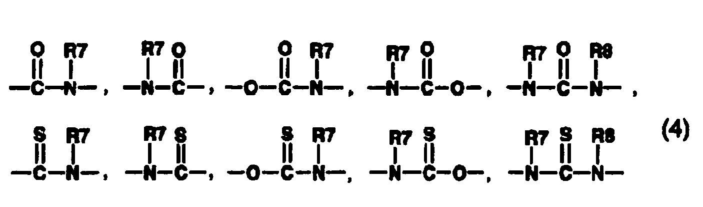 wherein R⁷ and R⁸ are independently hydrogen atom or an optionally substituted alkyl group having 1 to 4 carbons;
R¹ and R² are independently a hydrogen atom, a halogen atom, a hydroxyl group, an amino group, an alkyl group having 1 to 4 carbons, an alkoxy group having 1 to 4 carbons, an aminoalkyl group having 1 to 4 carbons, an alkylamino group having 1 to 4 carbons, an acyl group having 1 to 4 carbons, an acylamino group having 1 to 4 carbons, an alkylthio group having 1 to 4 carbons, a perfluoroalkyl group having 1 to 4 carbons, a perfluoroalkyloxy group having 1 to 4 carbons, a carboxyl group or an alkoxycarbonyl group having 1 to 4 carbons;
R³ is a hydroxyl or amino group, as well as isomers, diastereoisomers, enantiomers, tautomers and salts thereof, will overcome the disadvantages of the known single compounds.

It has now further surprisingly been found that the inventive combination, comprising at least one angiogenesis inhibitor and at least one HDAC inhibitor show a much better activity in comparison to the single compounds if applied alone.
The surprisingly better activity was especially found in a tumor model, as well as in a melanoma model, especially a human melanoma model.
Thus, the inventive synergistically effective combination allows the application of the medicament in a much more lower dosage, which results in a less dosage treatment for the patient.

Of special interest are those combinations comprising as compound A at least one compound from the group of compounds of general formula I, wherein
- X: is CH;
- W: is hydrogen;
- A, E and Q: independently of one another, are CH or N, whereby only a maximum of two nitrogen atoms are contained in the ring;
- R¹: is aryl or heteroaryl, which may be optionally substituted in one or more places in the same way or differently with hydrogen, halogen, hydroxy, C₁-C₁₂-alkyl, C₂-C₆-alkenyl, C₁-C₁₂-alkoxy, halo-C₁-C₆-alkyl, =O, -SO₂R⁶, -OR⁵, -SOR⁴, -COR⁶, -CO₂R⁶ or -NR⁷R⁸, whereby C₁-C₁₂-alkyl may be substituted with -OR⁵ or -NR⁷R⁸,
- R² and R³: independently of one another, are C₁-C₁₂ alkyl optionally substituted with -OR⁵; preferably C₁-C₂ alkyl optionally substituted with -OR⁵;
- R⁴: is C₁-C₁₂-alkyl, C₃-C₈-cycloalkyl, aryl or heteroaryl;
- R⁵: is hydrogen, C₁-C₁₂-alkyl, C₃-C₈-cycloalkyl or halo-C₁-C₆-alkyl;
- R⁶: is hydrogen, C₁-C₁₂-alkyl, C₃-C₈-cycloalkyl, halo-C₁-C₆-alkyl, aryl, or -NR⁷R⁸;
- R⁷ and R⁸,: independently of one another, are hydrogen, -SO₂R⁶, -COR⁶, aryl, C₃-C₈-cycloalkyl, C₁-C₁₂-alkyl, halo-C₁-C₁₂-alkyl, or C₁-C₁₂-alkoxy, whereby C₁-C₁₂-alkyl may be optionally substituted with -OR⁵ or -N(CH₃)₂, or R⁷ and R⁸ may also be chosen in such a way as to provide a 3-8 membered cycloalkyl ring, which may optionally contain further heteroatoms, such as nitrogen, oxygen or sulphur, and may be optionally substituted in one or more positions in the same way or differently with halogen, cyano, C₁-C₁₂-alkyl, C₁-C₁₂-alkoxy, halo-C₁-C₆-alkyl, =O, -OR⁵, COR⁶, -SR⁴, -SOR⁴ or -SO₂R⁶;
and as well as isomers, diastereoisomers, enantiomers, tautomers and salts thereof.

Of more interest are those combinations comprising as compound A at least one compound from the group of compounds of general formula I, wherein
- X: is CH;
- W: is hydrogen;
- A, E and Q: are each CH;
- R¹: is heteroaryl, optionally substituted in one or more places in the same way or differently with hydrogen, halogen, hydroxy, C₁-C₁₂-alkyl, C₂-C₆-alkenyl, C₁-C₁₂-alkoxy, halo-C₁-C₆-alkyl, =O, -SO₂R⁶, -OR⁵, -SOR⁴, -COR⁶, -CO₂R⁶ or -NR⁷R⁸, whereby C₁-C₁₂-alkyl may be substituted with -OR⁵ or -NR⁷R⁸,
- R² and R³,: independently of one another are C₁-C₂ alkyl;
- R⁴: is C₁-C₁₂-alkyl;
- R⁵: is hydrogen or -CH₃;
- R⁶: is C₁-C₁₂-alkyl or -NR⁷R⁸;
- R⁷ and R⁸: independently of one another, are hydrogen, -SO₂R⁶, -COR⁶, aryl, C₃-C₈-cycloalkyl, C₁-C₁₂-alkyl, halo-C₁-C₁₂-alkyl, or C₁-C₁₂-alkoxy, whereby C₁-C₁₂-alkyl may be optionally substituted with -OR⁵ or -N(CH₃)₂, or R⁷ and R⁸ may also be chosen in such a way as to provide a 4-7 membered cycloalkyl ring, which may optionally contain further heteroatoms, such as nitrogen, oxygen or sulphur, and may be optionally substituted in one or more positions in the same way or differently with halogen, cyano, C₁-C₁₂-alkyl, C₁-C₁₂-alkoxy, halo-C₁-C₆-alkyl, =O, -OR⁵, -COR⁶, -SR⁴, -SOR⁴ or -SO₂R⁶;
and as well as isomers, diastereoisomers, enantiomers, tautomers and salts thereof.

Of much more interest are those combinations comprising as compound A at least one compound from the group of compounds of general formula I, wherein
- X: is CH;
- W: is hydrogen;
- A, E and Q: are each CH;
- R¹: is the heteroaryl group 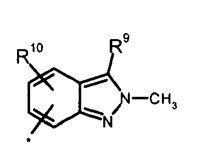 wherein R⁹ is hydrogen, halogen, C₁-C₁₂-alkyl, C₁-C₁₂-alkoxy, halo-C₁-C₆-alkyl, -COR⁶, -CO₂R⁶ or -NR⁷R⁸, whereby C₁-C₁₂-alkyl may be substituted with -OR⁵ or -NR⁷R⁸ and R¹⁰ is hydrogen or halogen;
- R² R³,:
- R⁴ and R⁶: independently of one another are -CH₃;
- R⁵: is hydrogen;
- R⁷ and R⁸: independently of one another, are hydrogen, -SO₂R⁶, -COR⁶, aryl, C₃-C₈-cycloalkyl, C₁-C₁₂-alkyl, halo-C₁-C₁₂-alkyl, or C₁-C₁₂-alkoxy, whereby C₁-C₁₂-alkyl may be optionally substituted with -OR⁵ or -N(CH₃)₂, or R⁷ and R⁸ may also be chosen in such a way as to provide a 5 or 6 membered cycloalkyl ring, which may optionally contain further heteroatoms, such as nitrogen, oxygen or sulphur, and may be optionally substituted in one or more positions in the same way or differently with halogen, cyano, C₁-C₁₂-alkyl, C₁-C₁₂-alkoxy, halo-C₁-C₆-alkyl, =O, -OR⁵ , -COR⁶, -SR⁴, -SOR⁴ or -SO₂R⁶;
and as well as isomers, diastereoisomers, enantiomers, tautomers and salts thereof.

Of selected interest are those combinations comprising as compound A at least one compound from the group of compounds of general formula I, wherein
- X: is CH;
- W: is hydrogen;
- A, E and Q: are each CH;
- R¹: is the heteroaryl group 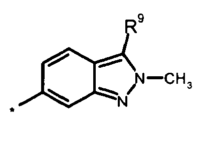 wherein R⁹ is hydrogen, halogen, C₁-C₁₂-alkyl, C₁-C₁₂-alkoxy, halo-C₁-C₆-alkyl, -COR⁶, -CO₂R⁶ or -NR⁷R⁸, whereby C₁-C₁₂-alkyl may be substituted with -OR⁵ or -NR⁷R⁸;
- R², R³,:
- R⁴ and R⁶: are each -CH₃;
- R⁵: is hydrogen;
- R⁷ and R⁸: independently of one another, are hydrogen, -COR⁶, -SO₂R⁶, C₁-C₁₂-alkyl;
and as well as isomers, diastereoisomers, enantiomers, tautomers and salts thereof.

Of most selected interest are those combinations comprising as compound A at least one compound from the group of compounds of general formula I, wherein
- X: is CH;
- W: is hydrogen;
- A, E and Q: are each CH;
- R¹: is the heteroaryl group 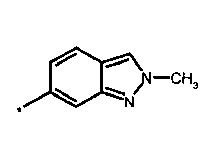
- R² and R³: are each -CH₃;
and as well as isomers, diastereoisomers, enantiomers, tautomers and salts thereof.

Selected examples of compounds according to general formula I (compound A) of the instant inventive combinations are as follows:

| | | | | |
|---|---|---|---|---|
| 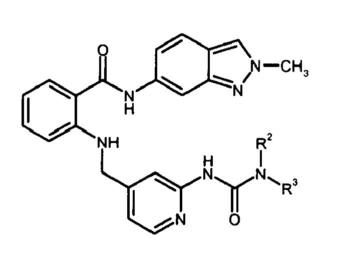 | | | | |

| **Example Nr.** | **R²** | **R³** | **MW** | **Mp. [°C] or MS (m/z)** |
|---|---|---|---|---|
| A-1 | -CH₃ | -CH₃ | | 184 |
| A-2 | -CH₂CH₃ | -CH₂CH₃ | 471.57 | Foam (ES+) 472 [M+H]⁺, 237 |
| A-3 | -CH₃ | -CH₂CH₂OH | 473.54 | Foam (ES+) 474 [M+H]⁺ |
| A-4 | -CH₃ | -CH₂CH₂OCH₃ | 487.57 | Mp.174 |
| A-5 | -CH₃ | -CH₂CH₃ | 457.54 | Foam (ES+) 458 [M+H]⁺, 230 |

| **Example Nr.** | **R¹** | **MW** | **Mp. [°C] or MS (m/z)** |
|---|---|---|---|
| A-6 | 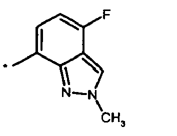 | 461.48 | Foam (ES+) 462 [M+H]⁺, 227, 222 |
| A-7 | 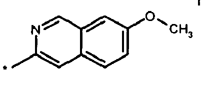 | 470.54 | Foam (ES+) 471 [M+H]⁺, 236 |
| A-8 | 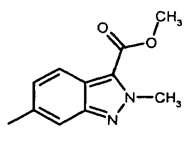 | | m/z (ES+) 502 [M+H]⁺ |
| A-9 | 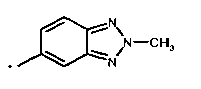 | 444.50 | Mp. 190.6 |
| A-10 | 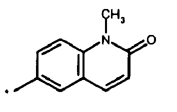 | 470.54 | (ES+) 471 [M+H]⁺, 236 |
| A-11 | 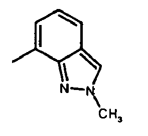 | 443.51 | Foam (ES+) 444 [M+H]⁺, 223 |
| A-12 | 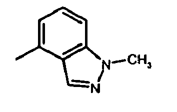 | 443.51 | Foam (ES+) 444 [M+H]⁺ |
| A-13 | 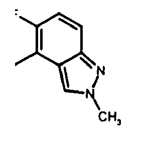 | 461.50 | Foam (ES+) 462 [M+H]⁺, 343, 252 |
| A-14 | 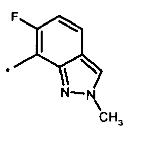 | 461.50 | Foam (ES+) 462 [M+H]⁺, 232 |
| A-15 | 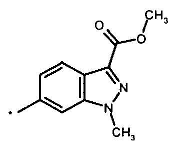 | 501.54 | Foam (ES+) 502 [M+H]⁺ |
| A-16 | 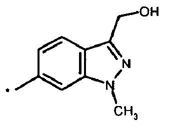 | 473.53 | Foam (ES+) 474 [M+H]⁺ |
| A-17 | 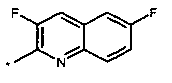 | 476.48 | Mp. 208 |
| A-18 | 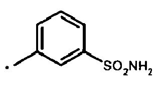 | 468.54 | Resin (ES+) 469 [M+H]⁺, 342 |
| A-19 | 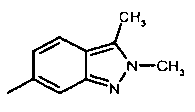 | | m/z (ES+) 458 [M+H]⁺, 230 |
| A-20 | 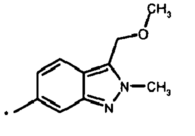 | 487.56 | Foam (ES+) 488 [M+H]⁺, 245 |
| A-21 | 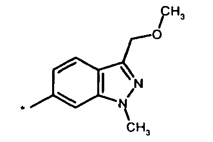 | 487.56 | Foam (ES+) 488 [M+H]⁺, 383, 247 |
| A-22 | 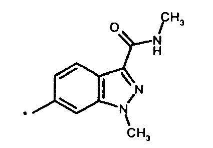 | 500.56 | Foam (ES+) 501 [M+H]⁺ |
| A-23 | 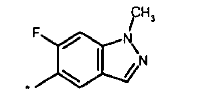 | 461.50 | Foam (ES+) 462 [M+H]⁺ |
| A-24 | 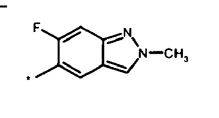 | 461.50 | Foam (ES+) 462 [M+H]⁺, 417 |
| A-25 | 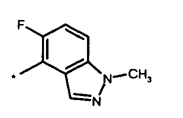 | 461.50 | Foam (ES+) 462 [M+H]⁺ |
| A-26 | 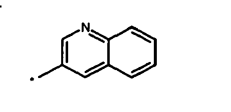 | 440.51 | Foam (ES+) 441 [M+H]⁺, 396, 221, 219 |
| A-27 | 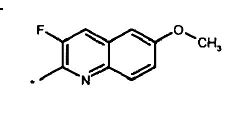 | 488.52 | Mp.211.6 |
| A-28 | 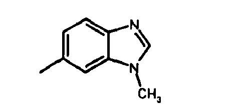 | 443.51 | Foam (ES+) 444 [M+H]⁺, 399, 222 |
| A-29 | 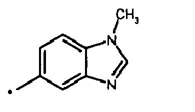 | 443.51 | Foam (ES+) 444 [M+H]⁺, 399, 223, 221 |
| A-30 | 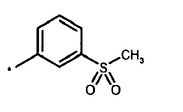 | 467.55 | Foam (ES+) 468 [M+H]⁺ |

| | | | |
|---|---|---|---|
| 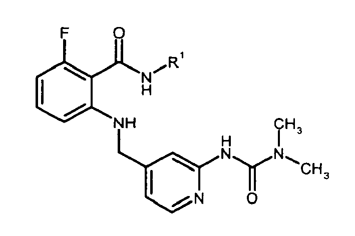 | | | |

| **Example Nr.** | **R¹** | **MW** | **Mp. [°C] or MS (m/z)** |
|---|---|---|---|
| A-31 | 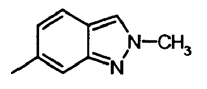 | | m/z (ES+) 462 [M+H]⁺ |

It should however be noted that these examples do not limit the inventive combination only to these compounds A.

The process for the production of the above mentioned compounds (A) is completely described in WO 2006/048251.

Of interest are those combinations wherein b) as compound B comprises at least one compound from the group of compounds of formula II), wherein n is an integer of 1 to 4.

Of special interest are those combinations wherein b) as compound B comprises at least one compound from the group of compounds of formula II), wherein n is an integer of 1 to 4.

Of further special interest are those combinations wherein b) as compound B comprises at least one compound from the group of compounds of general formula II), wherein Q is selected from the structures illustrated in formula (5): 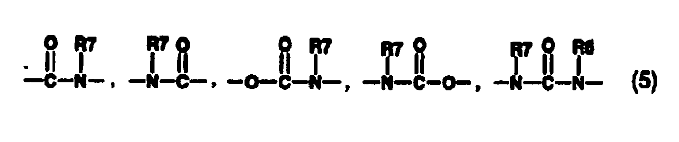 wherein R⁷ and R⁸ are as defined above.

Further of interest are those combinations wherein b) as compound B comprises at least one compound from the group of compounds of formula II), wherein A is an optionally substituted hetero ring, especially an optionally substituted pyridyl group.

Of special interest are also those combinations wherein b) as compound B comprises at least one compound from the group of compounds of of formula II), wherein n is 1 to 4; Q is selected from the structures illustrated in formula (5); A is an optionally substituted hetero ring, especially optionally substituted pyridyl group; most preferred, wherein X is direct bond, most preferred wherein R¹ and R² are a hydrogen atom, most preferred, wherein R³ is an amino group.

Also, of special interest are those combinations wherein b) as compound B comprises at least one compound from the group of compounds of of formula II), wherein Q is selected from the structures illustrated in formula (5); A is an optionally substituted hetero ring, especially optionally substituted pyridyl group; most preferred, wherein X is the structure represented by formula (6):

-(CH₂)e- (6)

wherein e is an integer of 1 to 4; most preferred wherein n is 1 and R¹ and R² are a hydrogen atom; most preferred, wherein R³ is an amino group.

Also of special interest are those combinations wherein b) as compound B comprises at least one compound from the group of compounds of formula II), wherein Q is selected from the structures illustrated in formula (5); A is an 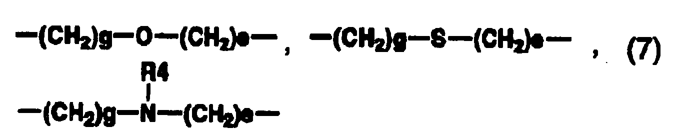 optionally substituted hetero ring, especially optionally substituted pyridyl group; most preferred, wherein X is selected from the structures illustrated in formula (7):
wherein e, g and R⁴ are as defined above; most preferred wherein n is 1 and R¹ and R² are a hydrogen atom; most preferred, wherein R³ is an amino group.

Interesting combinations wherein b) as compound B comprises at least one compound from the group of compounds of of formula II), wherein Q is selected from the structures illustrated in formula (5); A is an optionally substituted hetero ring, especially optionally substituted pyridyl group; most preferred, wherein X is selected from the structures illustrated in formula (8): 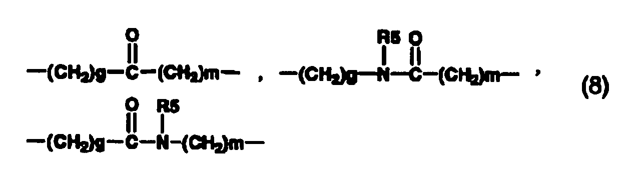 wherein g, m and R⁵ are as defined above; most preferred wherein n is 1 and R¹ and R² are a hydrogen atom; most preferred, wherein R³ is an amino group.

Of special interest are also those combinations, wherein b) as compound B comprises at least one compound from the group of compounds of formula II), wherein n is zero, and most preferred, Q is selected from the structures illustrated in formula (5); and most preferred, wherein A is an optionally substituted hetero ring; most preferred, wherein A is an optionally substituted pyridyl group; most preferred, wherein R¹ and R² are a hydrogen atom; most preferred, wherein R³ is an amino group.

Selected compounds of general formula II as compound B are for example the following compounds: 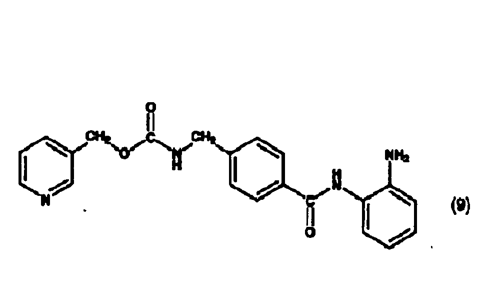 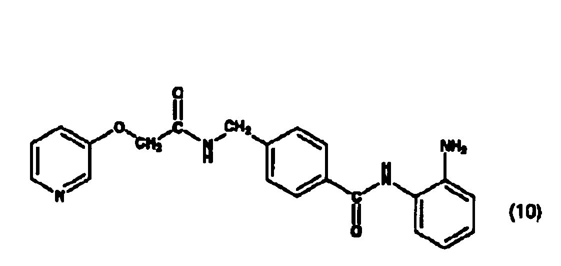 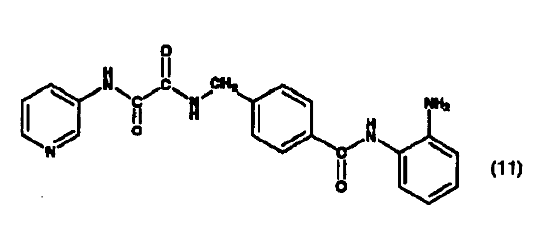 and

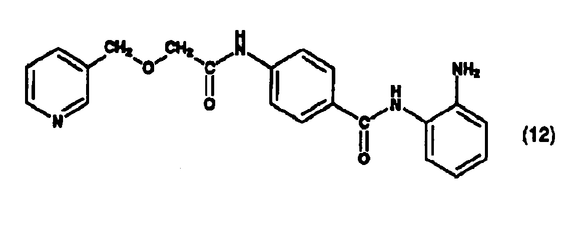

Of most interest is the combination of N'2'-{[2-(3,3-Dimethylureido)pyridine- 4-yl] methyl}-N-(2-methyl-2H-indazol-6-yl)Anthranilamid (ZK 261991), as compound A and 3-pyridylmethyl-N-{4-[(2-amino-phenyl)-carbamoyl]benzyl}carbamate (MS-275), as compound B in the treatment of cancer, tumors and melanoma.

The inventive combinations comprising at least one compound of formula (I), and at least one compound of formula II) can be used as a combined preparation simultaneously, separately or sequentially.

The invention further comprises the use of a combination for the manufacture of a medicament for a therapeutic application for treating cancer and tumors, wherein the compound(s) of formula (I), and the compound(s) of general formula II) are simultaneously, separately or sequentially used.

The inventive combinations can be used with at least one pharmaceutically acceptable diluent or carrier.

The invention also comprises a kit, comprising the inventive pharmaceutically active combination wherein the compound(s) of general formula (I) and compound(s) of general formula II), as a combined preparation are simultaneously, separately or sequentially used.

The inventive combinations can be used for enteral administration, such as nasal, buccal, rectal or, expecially, oral administration, and for parenteral administration, such as intravenous, intramuscular or subcutaneaous administration, to warm-blooded animals, especially, humans, are especially preferred. The compositions comprise the active ingredient alone or, preferably, together with a pharmaceutically acceptable carrier. The dosage of the active ingredient depends upon the disease to be treated and upon the species, gender, age, weight, and individual condition, the individual pharma-cokinetic data, and the mode of administration.

The inventive combinations can also used as a method for the prophylactic or especially therapeutic management of the human or animal body, to a process for the preparation thereof (especially in the form of compositions for the treatment of tumours) and to a method of treating tumour diseases, especially those mentioned hereinabove.

In the preferred embodiment, the pharmaceutical combinations is suitable for administration to a warm-blooded animal, especially humans or commercially useful mammals suffering form a disease responsive to an inhibition of angiogenesis or of VEGF-receptor tyrosine kinase, for example psoriasis or especially a neoplastic disease, and comprises an effective quantity of a compounds for the inhibition of angiogenesis or of VEGF-receptor tyrosine kinase, or a pharmaceutically acceptable salt thereof, if salt-forming groups are present, together with at least one pharmaceutically acceptable carrier.

The inventive combinations can be used for the prophylactic or especially therapeutic management of neoplastic and other proliferative diseases of a warm-blooded animal, especially a human or a commercially useful mammal requiring such treatment, especially suffering from such a disease.

The inventive combinations comprise from approximately 1 % to approximately 95% active ingredient, single-dose administration forms comprising in the preferred embodiment from approximately 5% to approximately 20% active ingredient. Unit dose forms are, for example, coated and uncoated tablets, ampoules, vials, suppositories or capsules. Further dosage forms are, for example, ointments, creams, pastes, foams, tinctures, lip-sticks, drops, sprays, dispersions, etc. Examples are capsules containing from about 0.05 g to about 1.0 g active ingredients.

The pharmaceutical combination of the present invention are prepared in a manner known *per se,* for example by means of conventional mixing, granulating, coating, dissolving or lyophilizing processes.

Preference is given to the use of solutions of the active ingredient, and also suspensions or dispersions, especially isotonic aqueous solutions, dispersions or suspensions which, for example in the case of lyophilized compositions comprising the active ingredients alone or together with a carrier, for example mannitol, can be made up before use. The pharmaceutical compositions may be sterilized and/or may comprise excipients, for example preservatives, stabilizers, wetting agents and/or emulsifiers, solubilizers, salts for regulating osmotic pressure and/or buffers and are prepared in a manner known *per se,* for example by means of conventional dissolving and lyophilizing processes. The said solutions or suspensions may comprise visosity-increasing agents, typically sodium carboxymethylcellulose, carboxymethylcellulose, dextran, polyvinylpyrrolidone, or gelatins, or also solubilizers, for example Tween 80 [polyoxyethylene(20)sorbitan mono-oleate; trademark of ICI Americas, Inc. USA].

Suspensions in oil comprise as the oil component the vegetable, synthetic, or semi-synthetic oils customary for injection purposes. In respect of such, special mention may be made of liquid fatty acid esters that contain as the acid component a long-chained fatty acid having from 8 to 22, expecially from 12 to 22, carbon atoms, for example lauric acid, tripdecylic acid, myristic acid, pentadecylic acid, palmitic acid, margaric acid, stearic acid, arachidic acid, behenic acid or corresponding unsaturated acids, for example oleaic acid, elaidic acid, erucic acid, brassidic acid or linoleic acid, if desired with the addition of anti-oxidants, for example vitamine E, β-carotene or 3,5-di-tert-butyl-4-hydroxytoluene. The alcohol component of these fatty acid esters has a maximum of 6 carbon atoms and is a monovalent or polyvalent, for example a mono-, di- or trivalent, alcohol, for example methanol, ethanol, propanol, butanol or pentanol or the isomers thereof, but especially glycol and glycerol. As fatty acid esters, therefore, the following are mentioned: ethyl oleate, isopropyl myristate, isopropyl palmitate, "Labrafil M 2375" (polyoxyethylene glycerol trioleate from Gattefossé, Paris), "Labrafil M 1944 CS" (unsaturated polyglycolized glycerides prepared by alcoholysis of apricot kernel oil and consisting of glycerides and polyethylene glycol ester; Gattefossé, France), "Labrasol" (saturated polyglycolized glycerides prepared by alcoholysis of TCM and consistitn of glycerides and polyethylene glycol ester; Gattefossé, France), and/or "Miglyol 812" (triglyceride of saturated fatty acids of chain length C₈ to C₁₂ from Hüls AG, Germany), but especially vegetable oils such as cottonseed oil, almond oil, olive oil, castor oil, sesame oil, soybean oil and more expecially groundnut oil.

The manufacture of injectable preparations is usually carried out under sterile conditions, as is filling, for example into ampoules or vials, and the sealing of the containers.

Pharmaceutical compositions for oral administration can be obtained, for example, by combining the active ingredient with one or more solid carriers, if desired granulating a resulting mixture, and processing the mixture of granules, if desired or necessary, by the inclusion of additional excipients, to form tablets or tablet cores.

Suitable carriers are especially fillers, such as sugars, for example lactose, saccharose, mannitol or sorbitol, cellulose preparations, and/or calcium phosphates, for example tricalcium phosphate or calcium hydrogen phosphate, and also binders, such as starches, for example corn, wheat, rice or potato starch, methylcellulose, hydroxypropyl methylcellulose, sodium carboxymethyl-cellulose, and/or polyvinylpyrrolidone, and/or, if desired, disintegrators, such as the above-mentioned starches, also carboxymethyl starch, crosslinked polyvinylpyrrolidone, alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

Tablet cores can be provided with suitable, optionally enteric, coatings through the use of, *inter alia,* condentrated sugar solutions, which may comprise gum arabic, talc, polyvinylpyrrolidone, polyethzlene glycol and/or titanium dioxide, or coating solutions in suitable organic solvents or solvent mixture, or for the preparation of enteric coatings, solutions of suitable cellulose preparations, such as acetly cellulose phthalate or hydroxypropylmethylcellulose phthalate. Dyes or pigments may be added to the tablets or tablet coatings, for example for identification purposes or to indicate different doses of active ingredient.

Pharmaceutical compostions for oral administration also include hard capsules consisting of gelatin, and also soft, sealed capsules consisting of gelatin and plasticizer, such as glycerol or sorbitol. The hard capsules may contain the active ingredient in the form of granules, for example in admixture with fillers, such as corn starch, binders, and/or glidants, such as talc or magnesium stearate, and optionally stabilizers. In soft capsules, the active ingredient is preferably dissolved or suspended in suitable liquid excipients, such as fatty oils, paraffin oil or liquid polyethylene glycols or fatty acid esters of ethylene or propylene glycol, to which stabilizers and detergents, for example of the polyoxyethylene sorbitan fatty acid ester type, may also be added.

Other oral dosage forms are, fior example syrups prepared in customary manner which comprise the active ingredient, for example, in suspended form and in a concentratin of about 5% to 20%, preferably about 10%, or in similar concentration that provides a suitable single dose, for example, when administered in measures of 5 or 10 ml. Also suitable are, for example, powdered or liquid concentrates for the preparation of shakes, for example in milk. Such concentrates may also be packaged in single-dose units.

Pharmaceutical compositions suitable for rectal administration are, for example, suppositories that consist of acombination of the active ingredient and a suppository base. Suitable suppository bases are, for example, naturral or synthetic triglycerides, paraffin hydrocarbons, polyethylene glycols or higher alkanols.

For prenteral administration, aqueous solutions of an active ingredient in water-soluble form, for example of a water-soluble salt, or aqueous injection suspensions that contain viscosity-increasing substances, for example sodium carboxymethylcellulose, sorbitol and/or dextran, and, if desired, stabilizers, are especially suitable. The active ingredient, optionally together with excipients, can also be in the form of a lyophilizate and can be made into a solution before parenteral administration by the addition of suitable solvents.

Solutions such as are used, for example, for parenteral administration can also be employed as infusion solutions.

Preferred preservatives are, for example, antioxidants, such as ascorbic acid, or micro-bicides, such as sorbic acid or benzoic acid.

The invention relates likewise to a process or a method for the treatment of one of the pathological conditions mentioned herineabove, especially a disease which responds to an inhibition of the VEGF-receptor tyrosine kinase or an inhibition of angiogenesis, especially a corresponding neoplastic disease or also psoriasis. The combination can be administered as such or especially in the form of pharmaceutical compositions, prophylactically or therapeutically, preferably in an amount effective against the said diseases, to a warm-blooded animal, for example a human, requiring such treatment. In case of an individual having a bodyweight of about 70 kg the daily dose administered is from approximately 0.1 g to approximately 5 g, preferably from approximately 0.5 g to approximately 2 g, of a compound of the present invention.

The present invention relates especially also to the use of the combination, as such or in the form of a pharmaceutical formulation with at least one pharmaceutically acceptable carrier for the therapeutic and also prophylactic management of one or more of the diseases mentioned hereinabove, especially a neoplastic disease or also psoriasis, more especially if the disease responds to an inhibition of angiogenesis or an inhibition of VEGF-receptor tyrosine kinase.

The present invention relates especially also to the use of the combination, as such or in the form of a pharmaceutical formulation with at least one pharmaceutically acceptable carrier for the therapeutic and also prophylactic management of one or more of the diseases mentione hereinabove, preferably a disease which responds to an inhibition of VGEF-receptor tyrosine kinase or an inhibition of angiogenesis, especially a neoplastic disease or also psoriasis, more especially if the said disease responds to an inhibition of VEGF-receptor tyrosine kinase or angiogenesis.

The present invention relates especially also to the use of the combination for the preparation of a pharmaceutical formulation for the therapeutic and also prophylactic managment of one or more of the diseases mentioned hereinabove, especially a neoplastic disease or also psoriasis, more especially if the disease responds to an inhibition of VEGF-receptor tyrosine kinase or angiogenesis.

The combination of this invention has differentiation-inducing effects and thus is useful as a therapeutic and/or improving combined agent to a variety of diseases such as malignant tumors, autoimmicro ne diseases, dermatologic diseases and parasitism.

As used herein, a "malignant tumor" includes hematologic malignancy such as acute leukemia, malignant lymphoma, micro ltiple myeloma and macroglobulinemia as well as solid tumors such as colon cancer, cerebral tumor, head and neck tumor, breast carcinoma, pulmonary cancer, esophageal cancer, gastric cancer, hepatic cancer, gallbladder cancer, bile duct cancer, pancreatic cancer, nesidioblastoma, renal cell carcinoma, adrenocortical cancer, urinary bladder carcinoma, prostatic cancer, testicular tumor, ovarian carcinoma, uterine cancer, chorionic carcinoma, thyroid cancer, malignant carcinoid tumor, skin cancer, malignant melanoma, osteogenic sarcoma, soft tissue sarcoma, neuroblastoma, Wilms tumor and retinoblastoma.

An autoimmicrone disease includes rheumatism, such as rheumatoide arthritis, diabetes, systemic lupus erythematodes, human autoimmicrone lymphocytotic lymphadenopathy, immicro noblastic lymphadenopathy, Crohn's disease and ulcerative colitis.

A dermatologic disease includes psoriasis, acne, eczema and atopic dermatitis.

Parasitism includes diseases such as malaria caused through vermination.

Further, the inventive combination can be used for the treatment haemeangioma, angiofribroma, diseases of the eyes, such as diabetic retinopathie, neovascular glaucoma, diseases of the kidney, such as glomerulonephritis, diabetic nephropatic diseases, malignant nephrosclerosis, thrombotic microangiopatic syndrome, disposes of transplants and glomerulopathy, fibrotic diseases, such as liver cirrhosis, mesangialic cell proliferative diseases and artheriosclerosis, injury of the nervous tissues, for inhibition of reocclusion of vascular systems after balloon catheter treatment, for artificial limbs, or after insert of mechanically devices for keeping open of vasculature, such as stents.
For the treatment of injury of the nervous tissues a rapid production of scars at the place of injury can be prevented. Thus, production of scars will be prevented before the axones can re-establish. Therefore a re-construction of nerves is possible.
Further, by using the inventive combination the ascites production within patients can be suppressed. Also, oedema resulted by VEGF can be suppressed.
Indications for the combination of this invention are not limited to these specific examples.

The active ingredient of the combination useful as a drug may be used in the form of a general pharmaceutical composition. The pharmaceutical composition maybe prepared with generally used diluents or excipients such as filler, extender, binder, moisturizing agent, disintegrator, surfactant and lubricant. The pharmaceutical composition may have a variety of dosage forms depending on its therapeutic purpose; typically tablet, pill, powder, solution, suspension, emulsion, granule, capsule, injection (e.g., solution, suspension) and suppository.

For preparing tablets, a variety of carriers well-known in the art may be used. Such a carrier includes excipients such as lactose, glucose, starch, calcium carbonate, kaoline, crystalline cellulose and silicic acid; binders such as water, ethanol, propanol, simple syrup, glucose solution, starch solution, gelatin solution, carboxymethyl cellulose, shellac, methyl cellulose and polyvinyl pyrrolidone; disintegrators such as dried starch, sodium alginate, powdered agar, calcium carmelose, starch and lactose; disintegration retarders such as sucrose, cocoa butter and hydrogenated oil; absorption promoters such as quaternary ammonium base and sodium lauryl sulfate; moisturizing agents such as glycerin and starch.; adsorbents such as starch, lactose, kaoline, bentonite, colloidal silicic acid; and glidants such as talc, stearates and polyethylene glycol. The tablet may be, if necessary, one coated with a common coating; for example, sugar-coated tablet, gelatin-coated tablet, enteric coated tablet, film-coated tablet, double-layer tablet and micro Itilayer tablet.

In forming pills, a variety of carriers well-known in the art may be used. Such a carrier includes excipients such as crystalline cellulose, lactose, starch, hydrogenated vegetable oil, kaoline and talc; binders such as powdered acacia, powdered tragacanth gum and gelatin; disintegrators such as calcium carmelose and agar.

Capsule may be prepared by blending an active ingredient with a variety of the above carriers as usual and filling the resulting blend into, for example, a hard or soft gelatin capsule or the like.

For preparing injection, solution, emulsion and suspension are sterilized and preferably isotonic with blood. It may be prepared using diluents commonly used in the art; for example, water, ethanol, macrogol, propylene glycol, ethoxylated isostearyl alcohol, polyoxyisostearyl alcohol and polyoxyethylene sorbitan fatty acid esters. The pharmaceutical preparation may contain sodium chloride necessary to prepare an isotonic solution, glucose or glycerin, as well as usual solubilizers, buffers and soothing agents.

Suppository may be formed using a variety of well-known carriers; for example, semi-synthetic glyceride, cocoa butter, higher alcohols, higher alcohol esters and polyethylene glycol.

Furthermore, the pharmaceutical combination may contain coloring agents, preservatives, perfumes, flavors, sweeteners and/or other drugs.

The amount of the active ingredient in the pharmaceutical combination of this invention may be, as appropriate, selected from a wide range with no limitations, and is generally about 1 to 70% by weight in the composition, preferably about 5 to 50% by weight.

An administration route of the pharmaceutical combination is not limited, and selected depending on patient's age, sex, severity of disease and other conditions. For example, tablet, pill, solution, suspension, emulsion, granule and capsule may be orally administered; injection may be intravenously administered solely or in combination with a common infusion fluid such as glucose, amino acids and the like, or if necessary, intramicro scularly, subcutaneously or intraperitoneally as a sole preparation. Suppository may be intrarectally administered.

Dose of the pharmaceutical combination of this invention may be selected, depending on their dosage form, patient's age, sex and severity of disease, and other conditions, as appropriate, but the amount of the active ingredient may be generally about 0.0001 to 100 mg/kg a day. It is recommended that a unit dosage form may contain about 0.001 to 1000 mg of the active ingredient.

The following examples describe the biological use of the inventive combination, however not restricting the scope of the invention to only these examples.

### Biological Examples

### Example 1

### Use of the Combination of N'2'-{[2-(3,3-Dimethylureido)pyridine-4-yl] methyl}-N-(2-methyl-2H-indazol-6-yl)Anthranilamid (ZK 261991) and 3-pyridylmethyl-N-{4-[(2-amino-phenyl)-carbamoyl]benzyl}carbamate (MS-275) in a human melanoma model

Human SK-Mel 28 melanoma cells were cultivated in RPMI1640 medium together with 1% glucose and 10% foetal calf serum. 3 million cells per animal have been transferred to NMRI nu/nu nude mice in a volume of 0,1ml in a 1:1 mixture consisting of media and matri gel. With achieving the tumor size of about 20mm², in this case on day 3, the treatment of the animals has been started.

For this, the animals heve been randomized and separated into the following test group (8 animals per group, each):
1. ZK 261991, 50mg/kg/day
2. MS-275; 10mg/kg/day
3. ZK261991 50mg/kg/day plus MS-275 10mg/kg/day
4. Untreated control (these animals received the solution solvent, only)
The compounds are orally daily applied via a probang till the end of the experiment on day 57. The tumor size was determined (length x width) after 6-7 days. On day 57, the animals were sacrified by breaking the neck and the tumors were isolated.The weight of the tumors was determined. The tumor size (tumor area in mm²) was inserted into a diagram, as function of the time. An average of the tumor weight of each group was calculated, as well as the variation and was inserted into a diagram. In the same time interval the tumor size was determined, also the body weight was determined by balancing. The data were inserted in a diagram as function of the time.

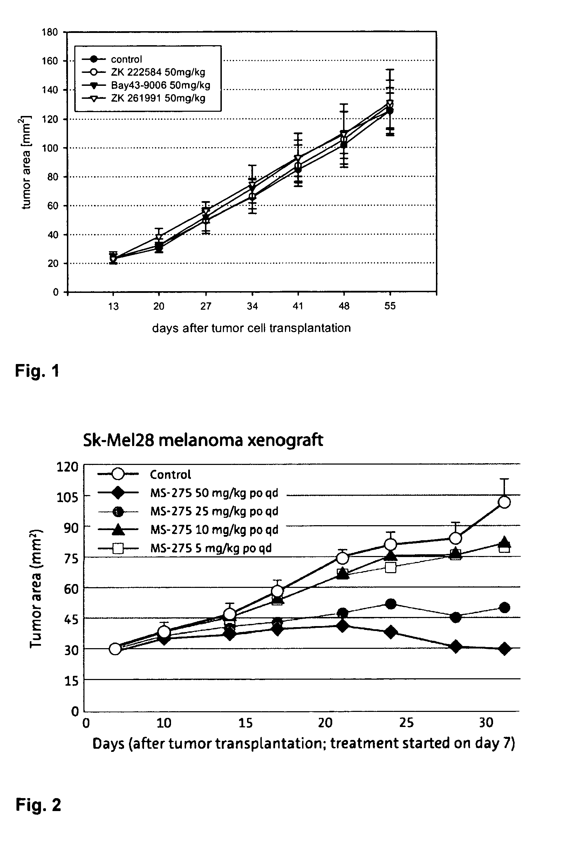

In a human melanoma model SK-Mel28 different angiogenesis inhibitors have been tested. No inhibitory effect onto the tumor growth could be determined (s. Fig.1). In this experiment the tumor have been daily treated with different compounds beginning after establishment of the tumors, respectively at a size of 20-30 mm². The compound ZK261991 was orally applied in a dosage of 50mg/ kg per day and in a volume of 0.1 ml/10g body weight, as a 5% DMSO/Ethanol 1:1 mixture in 0.085 Myrj85^{®}/0.9% Nacl formulation. ZK222584 (PTK/ZK, Vatalanib) comprised in the same formulation was applied. Further Bay43-9006 (Nexavar^{®}) in 30% hydroxy propyl cyclodextrine (HP-β-CD), pH 5.0 in the same volume of 0.1ml/10g body weight was applied. In a second independent experiment in the same melanoma model an effective reaction was found for the histone deacetylase inhibitor MS-275 at higher dosages of 25mg/kg and 50mg/kg. However, no reaction could be determined with a dosage of 5 and 10mg/kg (s. Fig.2). The tumors have been treated in a daily manner with different MS-275 dosages, beginning with the set up at a size of ca. 20-30mm². MS-275 was applied in 30% hydroxypropyl cyclodextrine (HP-β-CD), pH5.0, in a volume of 0.1 ml/10g body weight.

The results obtained from the two a. m. described examples show that a monotherapy with an angiogenesis inhibitor, respectively with an histone deacetylase inhibitor (depending on the dosage) lets to a small or no retardation of the tumor growth. Thus, a combination of an angiogenesis inhibitor and a histone deacetylase inhibitor was analysed in the human SK-Me128 melanoma model.

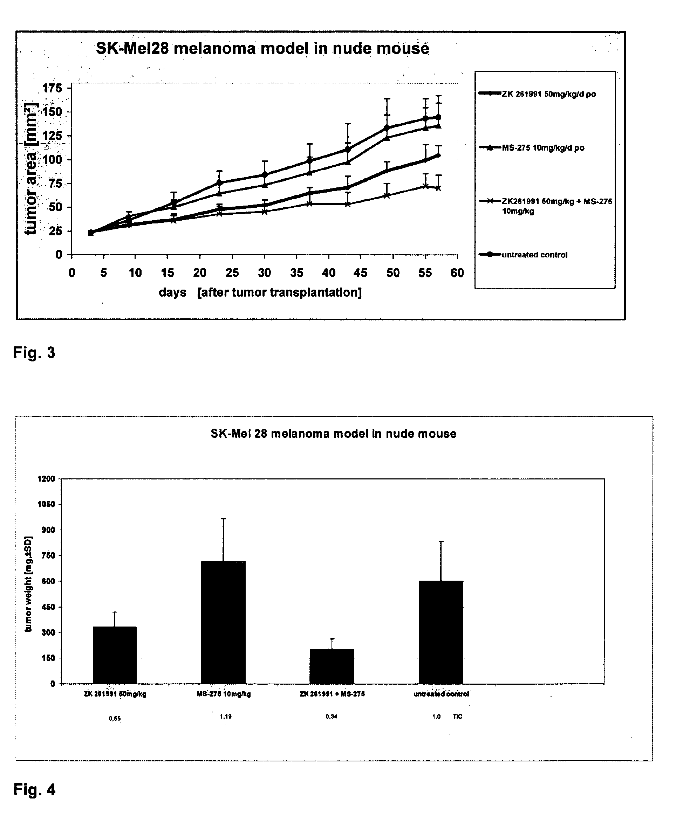

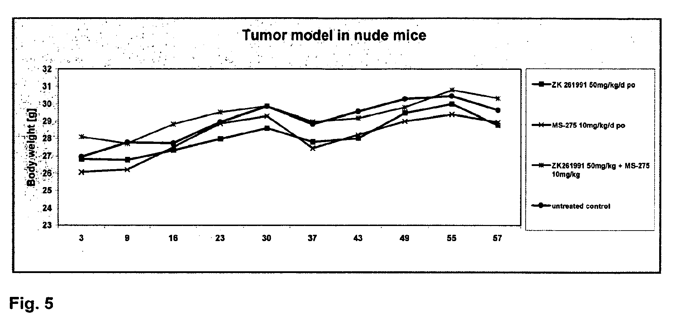

The tumors were treated in a daily manner with the different compounds beginning with the establishment, at a size of ca. 20.30mm². The treatment with the histonedeacetylase inhibitor MS-275 alone showed only a very small reaction at a dosage of 10mg/kg. The angiogenesis inhibitor ZK261991 showed a statistically significant but moderate result. However, the combination of both compounds let to a statistically significant reaction. It should be noted that the results of the combined compounds showed not only a statistically significant difference in comparison to the untreated control, but also to the both mono therapies (s. Fig. 3). For the calculation of the statistically signification the SigmaStat-programm with the One-Way-ANOVA-analysis was used. Table 1 shows the tumor areas of the single study groups on day 57, as well as the corresponding average and the standard deviation.
The analysis of the tumor weights shows the synergistically effect of the combination of both different inhibitors (s. Fig. 4). The combination lets to a 66% statistically significant reduction of the tumor weight of the treated group in comparison to the untreated control (the percentage of inhibition results from the converse T/C value, in the instant case 0.34). The mono therapy with MS-275 does not show any therapeutically effect (0% inhibition, and a more or less higher, statisticalle not very significant bigger tumor weight). The ZK261991 mono therapy let to a 45% reduction of the tumor weight in comparison to the untreated control group, respectively to a T/C value of 0.55. The addition of the effects of both monotherapies gives a max. reduction of 45%, which is clearly lower as the 66% reduction of the combination. Thus, the effect of the combination can be seen as synergistically. For the calculation of the statistically significance the SigmaStat-program with a One-Way-ANOVA-analysis was used. Table 2 shows the tumor weights of the single studied groups, the average and the standard deviation.
Theanalysis of the body weights did not show any signs of a acute toxicity (s. Fig.5).

**Table 1**

| **Tumor area of the single studied groups on day 57, average and standard deviation** | | | | |
|---|---|---|---|---|
| | **ZK 261991 50mg/kg** | **MS-275 10mg/kg** | **ZK 261991 and MS-275** | **Untreated control** |
| | 88,8 | 104,2 | 82,1 | 130,6 |
| | 116, 3 | 112,1 | 43, 3 | 140,1 |
| | 98,9 | 127,2 | 68,8 | 133,7 |
| | 109,5 | 132,1 | 72,7 | 129,8 |
| | 100,1 | 169,1 | 88,8 | *) |
| | 100,1 | 142,9 | 67,5 | 128,4 |
| | 105,0 | 128,6 | 62,6 | 190,1 |
| | 118,8 | 169,2 | 74,9 | 158,5 |
| **Average** | **104,7** | **135,7** | **70,1** | **144,5** |
| **standard deviation** | **9,9** | **23,8** | **13,7** | **22,7** |

| | | | | |
|---|---|---|---|---|
| *) animal died during experiment | | | | |

**Table 2**

| **Tumor area of the single studied groups on day 57, average and standard deviation** | | | | |
|---|---|---|---|---|
| | **ZK 261991 50mg/kg** | **MS-275 10mg/kg** | **ZK 261991 and MS-275** | **Untreated control** |
| | 262,7 | 479,2 | 305,9 | 220,8 |
| | 241,1 1 | 490 | 92,6 | 645,3 |
| | 49,9 | 610,9 | 162,5 | 485,5 |
| | 311,5 | 417,8 | 216,2 | 489,5 |
| | 261,2 | 969,4 | 237,3 | *) |
| | 295,9 | 1075 | 186,3 | 849,3 |
| | 377,1 | 733,1 | 237,5 | 563,2 |
| | 406,4 | 937,2 | 171,5 | 929,6 |
| **Average** | **331,6** | **714,1** | **201,2** | **597,6** |
| **standard deviation** | **88,3** | **253,4** | **63,5** | **239,2** |

| | | | | |
|---|---|---|---|---|
| *) animal died during experiment | | | | |

The results show a synergistic effect of the combination of ZK 261991/MS-275. There is clearly superiority over the single agents if applied alone, in contrast to the control example.

### Description of the Figures

**Fig. 1** shows the effect of different angiogenesis inhibitors on the growth of human SK-Mel28 melanoma cells in vivo.
**Fig. 2** shows the effect of the histone deacetylase inhibitor MS-275 on the growth of human SK-Mel28 melanoma cells in vivo.
**Fig. 3** shows the effect of the combination of the angiogenesis inhibitor ZK261991 together with the histone deacetylase inhibitor MS-275 on the growth of human SK-Mel28 melanoma cells in vivo.
**Fig. 4** shows the effect of the combination of the angiogenesis inhibitor ZK261991 together with the histone deacetylase inhibitor MS-275 on the tumor weight.
**Fig. 5** shows the effect of the combination of the angiogenesis inhibitor ZK261991 together with the histone deacetylase inhibitor MS-275 in comparism to the single compounds on the body weight.

## Claims

1. A combination comprising
a) as compound A
at least one compound from the group compounds of general formula I 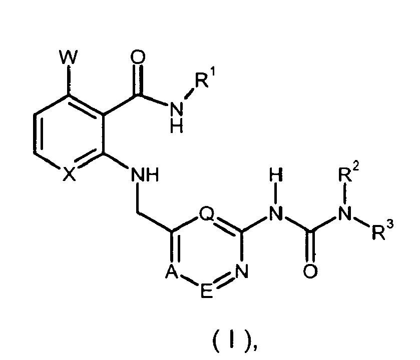 wherein
X is CH or N;
W is hydrogen or fluorine;
A, E and Q independently of one another, are CH or N, whereby only a maximum of two nitrogen atoms are contained in the ring;
R¹ is aryl or heteroaryl, which may be optionally substituted in one or more places in the same way or differently with hydrogen, halogen, hydroxy, C₁-C₁₂-alkyl, C₂-C₆-alkenyl, C₁-C₁₂-alkoxy, halo-C₁-C₆-alkyl, =O, -SO₂R⁶, -OR⁵, -SOR⁴, -COR⁶, -CO₂R⁶ or -NR⁷R⁸, whereby C₁-C₁₂-alkyl may be substituted with -OR⁵ or -NR⁷R⁸
R² and R³, independently of one another, are C₁-C₁₂ alkyl optionally substituted with -OR⁵;
R⁴ is C₁-C₁₂-alkyl, C₃-C₈-cycloalkyl, aryl or heteroaryl;
R⁵ is hydrogen, C₁-C₁₂-alkyl, C₃-C₈-cycloalkyl or halo-C₁-C₆-alkyl;
R⁶ is hydrogen, C₁-C₁₂-alkyl, C₃-C₈-cycloalkyl, halo-C₁-C₆-alkyl, aryl, or -NR⁷R⁸;
R⁷ and R⁸ independently of one another, are hydrogen, -SO₂R⁶, -COR⁶, aryl, C₃-C₈-cycloalkyl, C₁-C₁₂-alkyl, halo-C₁-C₁₂-alkyl, or C₁-C₁₂-alkoxy, whereby C₁-C₁₂-alkyl may be optionally substituted with -OR⁵ or -N(CH₃)₂, or R⁷ and R⁸ may also be chosen in such a way as to provide a 3-8 membered cycloalkyl ring, which may optionally contain further heteroatoms, such as nitrogen, oxygen or sulphur, and may be optionally substituted in one or more positions in the same way or differently with halogen, cyano, C₁-C₁₂-alkyl, C₁-C₁₂-alkoxy, halo-C₁-C₆-alkyl, =O, -OR⁵ , -COR⁶, -SR⁴,
-SOR⁴ or -SO₂R⁶;
and as well as isomers, diastereoisomers, enantiomers,
tautomers and salts thereof,
and
b) as compound B
at least one compound from the group of compounds of formula II 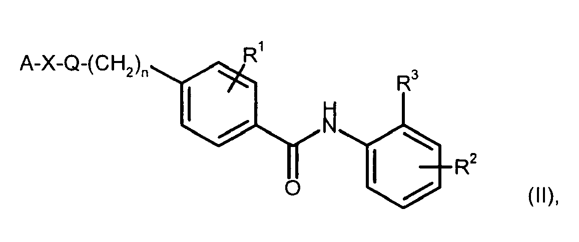 wherein
A is an optionally substituted phenyl group or an optionally substituted heterocyclic group wherein the substituent(s) for the phenyl group or the heterocyclic group is (are) 1 to 4 substituents selected from the group consisting of a halogen atom, a hydroxyl group, an amino group, a nitro group, a cyano group, an alkyl group having 1 to 4 carbons, an alkoxy group having 1 to 4 carbons, an aminoalkyl group having 1 to 4 carbons, an alkylamino group having 1 to 4 carbons, an acyl group having 1 to 4 carbons, an acylamino group having 1 to 4 carbons, an alkylthio group having 1 to 4 carbons, a perfluoroalkyl group having 1 to 4 carbons, a perfluoroalkyloxy group having 1 to 4 carbons, a carboxyl group, an alkoxycarbonyl group having 1 to 4 carbons, a phenyl group and a heterocyclic group;
X is a bond or a moiety having the following structure 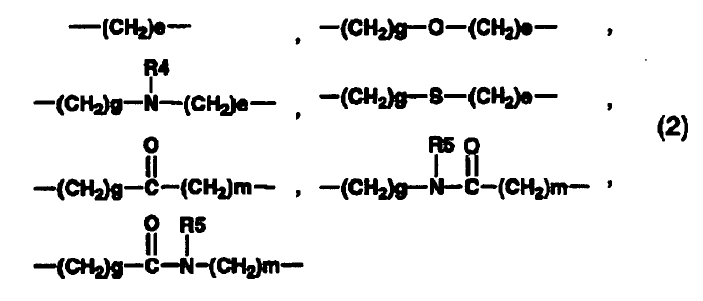 wherein e is an integer of 1 to 4; g and m are independently an integer of 0 to 4; R⁴ is a hydrogen atom or an optionally substituted alkyl group having 1 to 4 carbons, or the acyl group represented by formula (3) 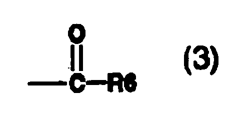 wherein R⁶ is an optionally substituted alkyl group having 1 to 4 carbons, a perfluoroalkyl group having 1 to 4 carbons, a phenyl group or a heterocyclic group; R⁵ is a hydrogen atom or an optionally substituted alkyl group having 1 to 4 carbons;
n is an integer of 0 to 4, provided that when X is a bond, n is not zero;
Q is a moiety having a structure selected from those illustrated in formula (4) 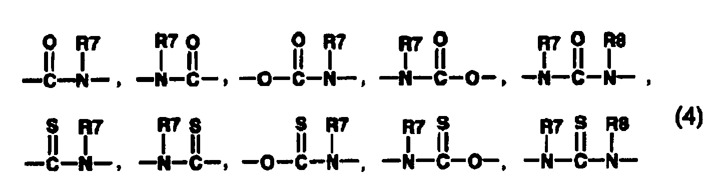 wherein R⁷and R⁸ are independently hydrogen atom or an optionally substituted alkyl group having 1 to 4 carbons;
R¹ and R² are independently a hydrogen atom, a halogen atom, a hydroxyl group, an amino group, an alkyl group having 1 to 4 carbons, an alkoxy group having 1 to 4 carbons, an aminoalkyl group having 1 to 4 carbons, an alkylamino group having 1 to 4 carbons, an acyl group having 1 to 4 carbons, an acylamino group having 1 to 4 carbons, an alkylthio group having 1 to 4 carbons, a perfluoroalkyl group having 1 to 4 carbons, a perfluoroalkyloxy group having 1 to 4 carbons, a carboxyl group or an alkoxycarbonyl group having 1 to 4 carbons;
R³ is a hydroxyl or amino group, as well as isomers, diastereoisomers, enantiomers, tautomers and salts thereof.

2. A combination according to claim 1, comprising at least one compound from the group of compounds of general formula I,
wherein
X is CH;
W is hydrogen;
A, E and Q independently of one another, are CH or N, whereby only a maximum of two nitrogen atoms are contained in the ring;
R¹ is aryl or heteroaryl, which may be optionally substituted in one or more places in the same way or differently with hydrogen, halogen, hydroxy, C₁-C₁₂-alkyl, C₂-C₆-alkenyl, C₁-C₁₂-alkoxy, halo-C₁-C₆-alkyl, =O, -SO₂R⁶, -OR⁵, -SOR⁴, -COR⁶, -CO₂R⁶ or -NR⁷R⁸, whereby C₁-C₁₂-alkyl may be substituted with -OR⁵ or -NR⁷R⁸
R² and R³ independently of one another, are C₁-C₁₂ alkyl optionally substituted with -OR⁵;
R⁴ is C₁-C₁₂-alkyl, C₃-C₈-cycloalkyl, aryl or heteroaryl;
R⁵ is hydrogen, C₁-C₁₂-alkyl, C₃-C₈-cycloalkyl or halo-C₁-C₆-alkyl;
R⁶ is hydrogen, C₁-C₁₂-alkyl, C₃-C₈-cycloalkyl, halo-C₁-C₆-alkyl, aryl, or -NR⁷R⁸;
R⁷ and R⁸, independently of one another, are hydrogen, -SO₂R⁶, -COR⁶, aryl, C₃-C₈-cycloalkyl, C₁-C₁₂-alkyl, halo-C₁-C₁₂-alkyl, or C₁-C₁₂-alkoxy, whereby C₁-C₁₂-alkyl may be optionally substituted with -OR⁵ or -N(CH₃)₂, or R⁷ and R⁸ may also be chosen in such a way as to provide a 3-8 membered cycloalkyl ring, which may optionally contain further heteroatoms, such as nitrogen, oxygen or sulphur, and may be optionally substituted in one or more positions in the same way or differently with halogen, cyano, C₁-C₁₂-alkyl, C₁-C₁₂-alkoxy, halo-C₁-C₆-alkyl, =O, -OR⁵ , -COR⁶, -SR⁴, -SOR⁴ or -SO²R⁶;
and as well as isomers, diastereoisomers, enantiomers,
tautomers and salts thereof.

3. A combination according to claims 1 or 2, comprising as compound A at least one compound from the group compounds of general formula I,
wherein
X is CH;
W is hydrogen;
A, E and Q are each CH;
R¹ is heteroaryl, optionally substituted in one or more places in the same way or differently with hydrogen, halogen, hydroxy, C₁-C₁₂-alkyl, C₂-C₆-alkenyl, C₁-C₁₂-alkoxy, halo-C₁-C₆-alkyl, =O, -SO₂R⁶, -OR⁵, -SOR⁴, -COR⁶, -CO₂R⁶ or -NR⁷R⁸, whereby C₁-C₁₂-alkyl may be substituted with -OR⁵ or -NR⁷R⁸;
R² and R³, independently of one another are C₁-C₂ alkyl;
R⁴ is C₁-C₁₂-alkyl;
R⁵ is hydrogen or -CH₃;
R⁶ is C₁-C₁₂-alkyl or -NR⁷R⁸;
R⁷ and R⁸ independently of one another, are hydrogen, -SO₂R⁶, -COR⁶, aryl, C₃-C₈-cycloalkyl, C₁-C₁₂-alkyl, halo-C₁-C₁₂-alkyl, or C₁-C₁₂-alkoxy, whereby C₁-C₁₂-alkyl may be optionally substituted with -OR⁵ or -N(CH₃)₂, or R⁷ and R⁸ may also be chosen in such a way as to provide a 4-7 membered cycloalkyl ring, which may optionally contain further heteroatoms, such as nitrogen, oxygen or sulphur, and may be optionally substituted in one or more positions in the same way or differently with halogen, cyano, C₁-C₁₂-alkyl, C₁-C₁₂-alkoxy, halo-C₁-C₆-alkyl, =O, -OR⁵ , COR⁶, -SR⁴, -SOR⁴ or -SO₂R⁶;
and as well as isomers, diastereoisomers, enantiomers,
tautomers and salts thereof.

4. A combination according to any one of claims 1 to 3, comprising as compound A at least one compound from the group compounds of general formula I, wherein
X is CH;
W is hydrogen;
A, E and Q are each CH;
R¹ is the heteroaryl group 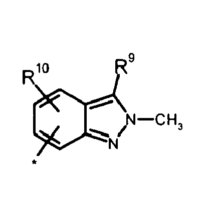 wherein R⁹ is hydrogen, halogen, C₁-C₁₂-alkyl, -C₁-C₁₂-alkoxy, halo-C₁-C₆-alkyl, -COR⁶, -CO₂R⁶ or -NR⁷R⁸, whereby C₁-C₁₂-alkyl may be substituted with -OR⁵ or -NR⁷R⁸ and R¹⁰ is hydrogen or halogen;
R², R³,
R⁴ and R⁶ independently of one another are -CH₃;
R⁵ is hydrogen;
R⁷ and R⁸ independently of one another, are hydrogen, -SO₂R⁶, -COR⁶, aryl, C₃-C₈-cycloalkyl, C₁-C₁₂-alkyl, halo-C₁-C₁₂-alkyl, or C₁-C₁₂-alkoxy, whereby C₁-C₁₂-alkyl may be optionally substituted with -OR⁵ or -N(CH₃)₂, or R⁷ and R⁸ may also be chosen in such a way as to provide a 5 or 6 membered cycloalkyl ring, which may optionally contain further heteroatoms, such as nitrogen, oxygen or sulphur, and may be optionally substituted in one or more positions in the same way or differently with halogen, cyano, C₁-C₁₂-alkyl, C₁-C₁₂-alkoxy, halo-C₁-C₆-alkyl, =O, -OR⁵ , -COR⁶, -SR⁴, -SOR⁴ or -SO₂R⁶;
and as well as isomers, diastereoisomers, enantiomers,
tautomers and salts thereof.

5. A combination according to any one of claims 1 to 4, comprising as compound A at least one compound from the group compounds of general formula I, wherein
X is CH;
W is hydrogen;
A, E and Q are each CH;
R¹ is the heteroaryl group 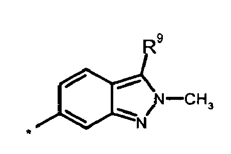 wherein R⁹ is hydrogen, halogen, C₁-C₁₂-alkyl, C₁-C₁₂-alkoxy, halo-C₁-C₆-alkyl, -COR⁶, -CO₂R⁶ or -NR⁷R⁸, whereby C₁-C₁₂-alkyl may be substituted with -OR⁵ or -NR⁷R⁸;
R², R³,
R⁴ and R⁶ are each -CH₃;
R⁵ is hydrogen;
R⁷ and R⁸ independently of one another, are hydrogen, -COR⁶, -SO₂R⁶, C₁-C₁₂-alkyl;
and as well as isomers, diastereoisomers, enantiomers, tautomers and salts thereof.

6. A combination according to any one of claims 1 to 5, comprising as compound A at least one compound from the group
compounds of general formula I,
wherein
X is CH;
W is hydrogen;
A, E and Q are each CH;
R¹ is the heteroaryl group 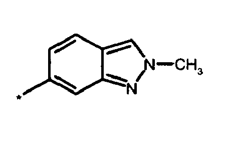
R² and R³ are each -CH₃;
and as well as isomers, diastereoisomers, enantiomers, tautomers and salts thereof.

7. A combination according to any one of claims 1 to 6,
comprising as compound A
N'2'-{[2-(3,3-Dimethylureido)pyridine-4-yl]methyl}-N-(2-methyl-2 H- indazol -6-yl)anthranilamid,
and
as compound B 3-pyridylmethyl-N-{4-[(2-amino-phenyl)-carbamoyl] benzyl}-carbamate.

8. A combination according to claim 1, comprising as compound B at least one compound from the group of compounds of formula II, wherein n is an integer of 1 to 4.

9. A combination according to claim 8, comprising as compound B at least one compound from the group of compounds of formula II, wherein Q is selected from the structures illustrated in formula (5): 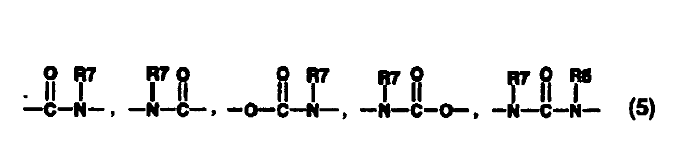 wherein R⁷ and R⁸ are as defined above.

10. A combination according to claim 9, comprising as compound B at least one compound from the group of compounds of formula II, wherein A is an optionally substituted hetero ring.

11. A combination according to claim 10, comprising as compound B at least one compound from the group of compounds of formula II, wherein A is an optionally substituted pyridyl group.

12. A combination according to claim 11, comprising as compound B at least one compound from the group of compounds of formula II), wherein X is a direct bond.

13. A combination according to claim 12, comprising as compound B at least one compound from the group of compounds of formula II, wherein R¹ and R² are a hydrogen atom.

14. A combination according to claim 13, comprising as compound B at least one compound from the group of compounds of formula II, wherein R³ is an amino group.

15. A combination according to claim 11, comprising as compound B at least one compound from the group of compounds of formula II, wherein X is the structure represented by formula (6):
-(CH₂)ₑ- (6)
wherein e is as defined above.

16. A combination according to claim 15, comprising as compound B at least one compound from the group of compounds of formula II, in which n is 1; and R¹ and R² are a hydrogen atom.

17. A combination according to claim 16, comprising as compound B at least one compound from the group of compounds of formula II, wherein additionally R³ is an amino group.

18. A combination according to claim 11, comprising as compound B at least one compound from the group of compounds of formula II, wherein X is selected from the structures illustrated in formula (7): 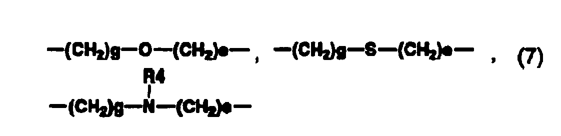 wherein e, g and R⁴ are as defined above.

19. A combination according to claim 21, comprising as compound B at least one compound from the group of compounds of formula II, wherein n is 1 and R¹ and R² are a hydrogen atom.

20. A combination according to claim 19, comprising as compound B at least one compound from the group of compounds of formula II, wherein R³ is an amino group.

21. A combination according to claim 11, comprising as compound B at least one compound from the group of compounds of formula II, wherein X is selected from the structures illustrated in formula (8): 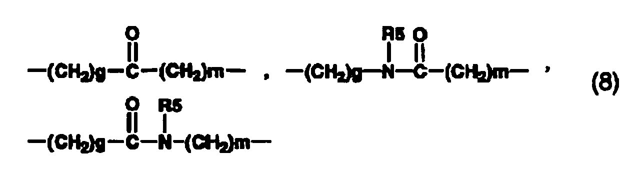 wherein g, m and R⁵ are as defined above.

22. A combination according to claim 21, comprising as compound B at least one compound from the group of compounds of formula II, wherein n is 1; and R¹ and R² are a hydrogen atom.

23. A combination according to claim 22, comprising as compound B at least one compound from the group of compounds of formula II, wherein R³ is an amino group.

24. A combination according to claim 1, comprising as compound B at least one compound from the group of compounds of formula II, wherein n is zero.

25. A combination according to claim 24, comprising as compound B at least one compound from the group of compounds of formula II, wherein Q is selected from the structures illustrated in formula (5): 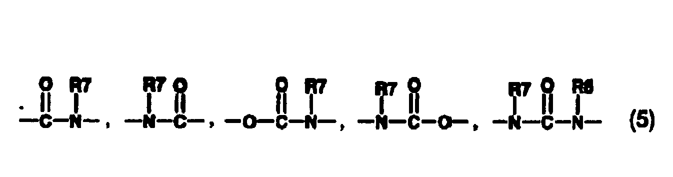 wherein R⁷ and R⁸ are as defined above.

26. A combination according to claim 25, comprising as compound B at least one compound from the group of compounds of formula II, wherein A is an optionally substituted pyridyl group, and wherein R¹ and R² are a hydrogen atom, and wherein R³ is an amino group.

27. A combination according to claim 1, comprising as compound B at least one compound of the following structure: 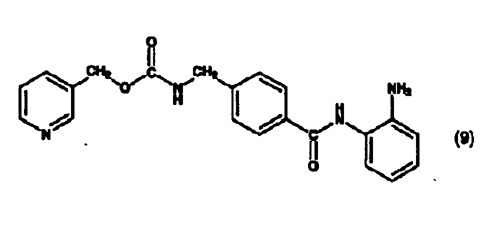 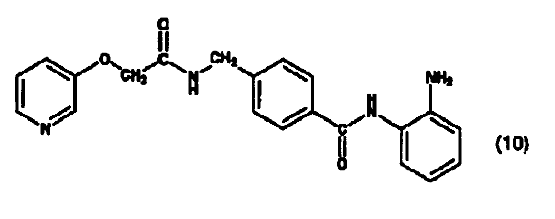 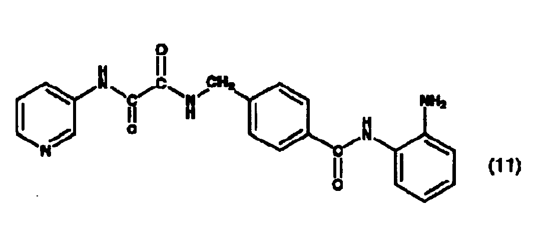 and

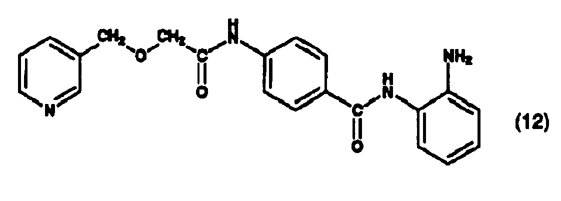

28. A combination according to any one of claims 1 to 27, wherein at least one compound of formula I, and at least one compound of formula II can be used as a combined preparation simultaneously, separately or sequentially.

29. Use of a combination according to any one of claims 1 to 27, for the manufacture of a medicament for a therapeutic application for treating cancer, tumors and melanoma, wherein the compound(s) of formula I, and compound(s) of general formula II are simultaneously, separately or sequentially used.

30. Use according to claim 29, with at least one pharmaceutically acceptable diluent or carrier.

31. A kit, comprising the combination according to any one of claims 1 to 27, wherein the compound(s) of general formula I and compound(s) of general formula II as a combined preparation are simultaneously, separately or sequentially used.

32. Use of the combination according to any one of claims 1 to 27, for the treatment of haemeangioma, angiofribroma, diseases of the eyes, such as diabetic retinopathie, neovascular glaucoma, diseases of the kidney, such as glomerulonephritis, diabetic nephropatic diseases, malignant nephrosclerosis, thrombotic microangiopatic syndrome, disposes of transplants and glomerulopathy, fibrotic diseases, such as liver cirrhosis, mesangialic cell proliferative diseases and artheriosclerosis, injury of the nervous tissues, for inhibition of reocclusion of vascular systems after balloon catheter treatment, for artificial limbs, or after insert of mechanically devices for keeping open of vasculature, such as stents.

33. Use of the combination according to any of claims 1 to 27, for the treatment of injury of the nervous tissues.

34. Use of the combination according to any one of claims 1 to 27, for the suppression of oedema resulted by VEGF.

35. A combination according to any one of claims 1 to 27, for use in a method for the treatment of the human or animal body.

36. A combination according to any one of claims 1 to 27, together with at least one pharmaceutically acceptable carrier, or excipient.

37. Use of a combination according to any one of claims 1 to 27, for the preparation of a pharmaceutical product for the treatment of a disease which responds to an inhibition of angiogenesis.

38. Use of a combination according to any one of claims 1 to 27, for the preparation of a pharmaceutical product for the treatment of a disease which responds to an inhibition of VEGF-receptor tyrosine kinase.

39. Use of a combination according to any one of claims 1 to 27, for the treatment of a disease wich responds to an inhibition of VEGF-receptor kinase.

40. Use of a combination according to any one of claims 1 to 27, which is suitable for administration to a warm-blooded animal, especially suffering from a disease which responds to an inhibition of angiogenesis or of VEGF-receptor tyrosine kinase, comprising an effective quantity of the combination, together with at least one pharmaceutically acceptable carrier.

41. A method for treatment of disease which responds to an inhibition of VEGF-receptor tyrosine kinasse or an inhibition of angiogenesis, which comprises administering a combination according to any one of claims 1 to 27, in a compound quantity effective against the said diseases, to a warm-blooded animal requiring such treatment in a compound quantity suitable for the treatment of the said disease.
